# EUROPEAN PATENT APPLICATION

(11) **EP 4 501 123 A1**
(43) Date of publication of application: **05.02.2025**
(21) Application number: 23781034.6
(22) Date of filing: 31.03.2023
(51) Int. Cl.: A23F 3/16, A23F 5/24, A23J 3/14, A61P 37/08, A61K 9/107, A61K 36/48, A23L 9/20, A23D 7/00, A23D 7/005, A61K 38/02, A23C 11/10, A23L 11/60, A23L 11/65, A23L 5/00, A23L 23/00, A23L 29/10, A61K 8/06, A61K 8/64, A61K 8/9789, C09K 23/30

(54) **PROTEIN, PROTEIN PARTICLE DISPERSION AND EMULSION COMPOSITION INCLUDING SAID PROTEIN, AND MANUFACTURING METHODS THEREOF**

(30) Priority: 31.03.2022 JP 2022060926
(71) Applicant: Mitsubishi Chemical Corporation, Tokyo 100-8251 (JP)
(72) Inventor: HANASAKI, Minako, Tokyo 100-8251 (JP); TANAKA, Toshiyuki, Tokyo 100-8251 (JP)
(74) Representative: Ter Meer Steinmeister & Partner
(86) International application number: PCT/JP2023/013462
(87) International publication number: WO 2023/191026

(57) **Abstract**

One problem addressed by the present invention is to provide an emulsion composition that maintains its quality even when stored frozen. The problem is solved by a protein that satisfies one of the following conditions (A) to (C) and is partially in the state of particles when made into an aqueous dispersion: (A): having at least one peak in a region of a molecular weight of 66,000 or more in terms of polyethylene glycol in a gel filtration chromatography elution curve; (B): having a weight-average molecular weight of more than 27,000 in terms of polyethylene glycol as measured by gel filtration chromatography; and (C): having a number-average molecular weight of more than 3,500 in terms of polyethylene glycol as measured by gel filtration chromatography.

## Description

### TECHNICAL FIELD

The present invention relates to: a protein; a protein particle dispersion and an emulsion composition, which contain the protein; protein particles; an emulsion composition containing the protein particles; production methods of the same; and a food, an animal-derived food substitute, a pharmaceutical product, a cosmetic product, and a personal care product, which contain the same.

### BACKGROUND ART

In the field of foods, emulsification with a surfactant has been utilized conventionally. Emulsification with a surfactant is thermodynamically unstable; therefore, in order to ensure long-term stability as well as stability against high-temperature sterilization process, it is necessary to, for example, reduce the oil-droplet particle size of an oil-in-water (O/W) emulsion to the sub-micron level.

In recent years, in the field of foods, there is an increasing need for appetizing appearances, flavors (which stimulate the senses of taste and smell), and textures, as well as interests in ingredients driven by health consciousness. Accordingly, it is demanded to develop an oil-in-water emulsion having an emulsion size and a structure that are different from those of conventional emulsion compositions using surfactants.

As a method other than emulsification with a surfactant, it is known that an emulsion can be stabilized using microparticles of a colloid or the like. An emulsion stabilized by adsorption of microparticles to the interface of water and oil (e.g., an organic solvent or an oil-and-fat) is referred to as "microparticle-stabilized emulsion" or "Pickering emulsion". In recent years, microparticle-stabilized emulsions (Pickering emulsions) have been actively studied.

In the field of foods, for example, the method of Non-patent Document 1 has been disclosed.

### RELATED ART DOCUMENT

### NON-PATENT DOCUMENT

[Non-Patent Document 1] Shuning Zhang et al., Food Hydrocolloids 2020, 102, 105583

### SUMMARY OF THE INVENTION

### PROBLEMS TO BE SOLVED BY THE INVENTION

In the food applications, however, the market demands a higher level of quality, and there is a need for an emulsion composition that satisfies such demand. In recent years, the demand for frozen foods has increased due to the COVID-19 pandemic, and there is an increasing demand for not only heat resistance in cooking and heating processes, but also storage stability in freezing and thawing. An emulsion composition having a high freezing and thawing resistance is extremely useful since it removes the constraint of having to transport an emulsion composition in a frozen state; however, it is technically highly difficult to produce such an emulsion composition.

In view of the above, a first object of the present invention is to provide an emulsion composition that maintains its quality even when stored frozen. A second object of the present invention is to provide an emulsion composition that withstands freezing and thawing. A third object of the present invention is to provide an emulsion composition that has a combination of freezing resistance, thawing resistance, cooling resistance, and heat resistance. A fourth object of the present invention is to improve the quality of taste (provide richness), which is difficult in a conventional emulsion using a surfactant.

Further, a fifth object of the present invention is to provide an emulsion that can be a substitute for an edible material containing a specific allergenic substance, depending on the allergenic substance of the consumer. A sixth object of the present invention is to provide an animal-derived food substitute containing no animal-derived ingredients, which contributes to a reduction in environmental load that is a social issue.

### MEANS FOR SOLVING THE PROBLEMS

The present inventors intensively studied to solve the above-described problems and consequently discovered that the problems can be solved by using a protein satisfying specific conditions in an emulsion composition. In other words, the gist of the present invention resides in the following.
[1] A protein, satisfying one of the following conditions (A) to (C) and being partially in the state of particles when made into an aqueous dispersion:
   (A): having at least one peak in a region of a molecular weight of 66,000 or more in terms of polyethylene glycol in a gel filtration chromatography elution curve;
   (B): having a weight-average molecular weight of more than 27,000 in terms of polyethylene glycol as measured by gel filtration chromatography; and
   (C): having a number-average molecular weight of more than 3,500 in terms of polyethylene glycol as measured by gel filtration chromatography.
[2] The protein according to [1], satisfying at least two of the conditions (A) to (C).
[3] The protein according to [1] or [2], wherein a raw material protein constituting the protein is a plant protein.
[4] The protein according to [3], wherein the plant protein is a protein derived from a Fabaceae plant.
[5] The protein according to [4], wherein the Fabaceae plant is a *Phaseolus* plant, a *Cicer* plant, a *Pisum* plant, a *Lens* plant, or a *Lupinus* plant.
[6] The protein according to any one of [1] to [5], wherein the particles in the aqueous dispersion have an average particle size of 0.005 µm to 10 µm.
[7] A protein particle dispersion, containing the protein according to any one of [1] to [6].
[8] A method of producing the protein according to any one of [1] to [6], the method including the following steps (1) and (2):
   Step (1): the step of mixing water and a raw material protein to prepare a mixture; and
   Step (2): the step of heat-treating the mixture at 60°C to 200°C for 1 second to 120 minutes.
[9] A method of producing a protein, the method including the following steps (1) and (2') and the protein being partially in the state of particles when made into an aqueous dispersion,
   wherein the below-described raw material protein is a plant protein:
   Step (1): the step of mixing water and a raw material protein to prepare a mixture; and
   Step (2'): the step of heat-treating the mixture at 60°C to 200°C for 1 second to 120 minutes under a pressure other than normal pressure.
[10] An emulsion composition, containing water, an oil-and-fat, and a protein,
   wherein
   the protein exists in an interface between the water and the oil-and-fat, and
   the protein is the protein according to any one of [1] to [6].
[11] The emulsion composition according to [10], wherein the oil-and-fat is an edible oil-and-fat.
[12] The emulsion composition according to [10] or [11], which is an oil-in-water emulsion composition.
[13] A method of producing an emulsion composition, the method including the steps of:
   dispersing the protein according to any one of [1] to [6] in water to obtain an aqueous dispersion of protein particles; and
   mixing the thus obtained aqueous dispersion of protein particles with an oil-and-fat, and stirring the resulting mixture.
[14] A method of producing an emulsion composition, the method including:
   a step of dispersing the protein according to any one of [1] to [6] in an oil-and-fat to obtain an oil-and-fat dispersion of protein particles; and
   a step of mixing the thus obtained oil-and-fat dispersion of protein particles with water, and stirring the resulting mixture.
[15] The emulsion composition according to any one of [10] to [12], which is for a food.
[16] A food, containing the protein according to any one of [1] to [6].
[17] A food, containing the emulsion composition according to any one of [10] to [12].
[18] An animal-derived food substitute, containing the protein according to any one of [1] to [6].
[19] An animal-derived food substitute, containing the emulsion composition according to any one of [10] to [12].
[20] A milk substitute, containing the protein according to any one of [1] to [6].
[21] A milk substitute, containing the emulsion composition according to any one of [10] to [12].
[22] A pharmaceutical product, containing the protein according to any one of [1] to [6].
[23] A pharmaceutical product, containing the emulsion composition according to any one of [10] to [12].
[24] A cosmetic product, containing the protein according to any one of [1] to [6].
[25] A cosmetic product, containing the emulsion composition according to any one of [10] to [12].
[26] A personal care product, containing the protein according to any one of [1] to [6].
[27] A personal care product, containing the emulsion composition according to any one of [10] to [12].
[28] Protein particles, satisfying one of the following conditions (A) to (C) when made into an aqueous dispersion:
   (A): having at least one peak in a region of a molecular weight of 66,000 or more in terms of polyethylene glycol in a gel filtration chromatography elution curve;
   (B): having a weight-average molecular weight of more than 27,000 in terms of polyethylene glycol as measured by gel filtration chromatography; and
   (C): having a number-average molecular weight of more than 3,500 in terms of polyethylene glycol as measured by gel filtration chromatography.
[29] The protein particles according to [28], satisfying at least two selected from the conditions (A) to (C).
[30] Protein particles, produced by a production method including:
   a step of mixing water and a raw material protein to prepare a mixture; and
   a step of heat-treating the mixture at 60°C to 200°C for 1 second to 120 minutes.
[31] The protein particles according to any one of [28] to [30], wherein a raw material protein constituting the protein particles is a plant protein.
[32] The protein particles according to any one of [28] to [31], wherein the raw material protein constituting the protein particles is a protein derived from a Fabaceae plant.
[33] The protein particles according to [32], wherein the Fabaceae plant is a *Phaseolus, Cicer, Pisum, Lens,* or *Lupinus* plant.
[34] The protein particles according to any one of [28] to [33], having an average particle size of 0.005 µm to 10 µm.
[35] A method of producing the protein particles according to any one of [28], [29], and [31] to [34], the method including:
   a step of mixing water and a raw material protein to prepare a mixture; and
   a step of heat-treating the mixture at 60°C to 200°C for 1 second to 120 minutes.
[36] An emulsion composition, containing water, an oil-and-fat, and protein particles,
   wherein
   the protein particles exist in an interface between the water and the oil-and-fat, and
   the protein particles are the protein particles according to any one of [28] to [34].
[37] The emulsion composition according to [36], wherein the oil-and-fat is an edible oil-and-fat.
[38] The emulsion composition according to [36] or [37], which is an oil-in-water emulsion composition.
[39] A method of producing an emulsion composition, the method including:
   a step of dispersing the protein particles according to any one of [28] to [34] in water; and
   a step of mixing the thus obtained aqueous dispersion of the protein particles with an oil-and-fat, and stirring the resulting mixture.
[40] A method of producing an emulsion composition, the method including:
   a step of dispersing the protein particles according to any one of [28] to [34] in an oil-and-fat; and
   a step of mixing the thus obtained oil-and-fat dispersion of the protein particles with water, and stirring the resulting mixture.
[41] The emulsion composition according to any one of [36] to [38], which is for a food.
[42] A food, containing the protein particles according to any one of [28] to [34].
[43] A food, containing the emulsion composition according to any one of [36] to [38].
[44] An animal-derived food substitute, containing the protein particles according to any one of [28] to [34].
[45] An animal-derived food substitute, containing the emulsion composition according to any one of [36] to [38].
[46] A pharmaceutical product, containing the protein particles according to any one of [28] to [34].
[47] A cosmetic product, containing the protein particles according to any one of [28] to [34].
[48] A personal care product, containing the protein particles according to any one of [28] to [34].
[49] A pharmaceutical product, containing the emulsion composition according to any one of [36] to [38].
[50] A cosmetic product, containing the emulsion composition according to any one of [36] to [38].
[51] A personal care product, containing the emulsion composition according to any one of [36] to [38].

### EFFECTS OF THE INVENTION

According to the present invention, an emulsion composition that maintains its quality even when stored frozen can be provided. In addition, an emulsion composition that withstands freezing and thawing can be provided. Further, an emulsion composition that has a combination of freezing resistance, thawing resistance, cooling resistance, and heat resistance can be provided. Moreover, an emulsion composition having a higher quality of taste than conventional emulsion compositions can be provided.

### BRIEF DESCRIPTION OF THE DRAWINGS

[FIG. 1] FIG. 1 shows a gel filtration chromatography elution curve of the protein particle aqueous dispersion A1-1 that was obtained in Example 1.
[FIG. 2] FIG. 2 shows a gel filtration chromatography elution curve of the protein particle aqueous dispersion B1-1 that was obtained in Example 2.
[FIG. 3] FIG. 3 shows a gel filtration chromatography elution curve of the protein particle aqueous dispersion C1-1 that was obtained in Comparative Example 1.
[FIG. 4] FIG. 4 shows a gel filtration chromatography elution curve of the protein particle aqueous dispersion D1-1 that was obtained in Comparative Example 2.
[FIG. 5] FIG. 5 shows a gel filtration chromatography elution curve of the protein particle aqueous dispersion E1-1 that was obtained in Comparative Example 3.
[FIG. 6] FIG. 6 shows a gel filtration chromatography elution curve of the protein particle aqueous dispersion F1 that was obtained in Comparative Example 4.

### MODE FOR CARRYING OUT THE INVENTION

Embodiments of the present invention will now be described in detail. The following descriptions of requirements are merely examples (representative examples) of the embodiments of the present invention, and the present invention is not limited thereto within the scope of the gist of the present invention. The term "protein particles" used herein refers to granulated substaces containing a protein as a main component.

### <Protein Particles>

A first embodiment of the present invention will now be described.

The first embodiment of the present invention provides protein particles satisfying one of the following conditions (A) to (C) when made into an aqueous dispersion:
(A): having at least one peak in a region of a molecular weight of 66,000 or more in terms of polyethylene glycol in a gel filtration chromatography elution curve;
(B): having a weight-average molecular weight of more than 27,000 in terms of polyethylene glycol as measured by gel filtration chromatography; and
(C): having a number-average molecular weight of more than 3,500 in terms of polyethylene glycol as measured by gel filtration chromatography.

The protein particles preferably satisfy at least two selected from the conditions (A) to (C), more preferably satisfy all of the three conditions (A) to (C).

Although a detailed mechanism is not clear, when protein particles satisfying at least one of the above-described conditions are incorporated into an emulsion composition, since the protein particles have appropriate physical properties in terms of "wettability that allows stabilization of an interface between water and an oil-and-fat", "size", "relaxation ability of particles to withstand a change in the state of a liquid phase", and the like, an emulsion composition that maintains its quality even when stored frozen can be obtained.

In an aqueous dispersion of the protein particles, the concentration of the protein particles is usually 0.001% by mass or higher, preferably 0.002% by mass or higher, more preferably 0.005% by mass or higher, still more preferably 0.01% by mass or higher, especially preferably 0.05% by mass or higher, particularly preferably 0.1% by mass or higher, specially preferably 0.5% by mass or higher, most preferably 1% by mass or higher, particularly most preferably 2% by mass or higher, exceptionally preferably 4% by mass or higher.

Meanwhile, the concentration of the protein particles is usually 50% by mass or lower, preferably 45% by mass or lower, more preferably 40% by mass or lower, still more preferably 35% by mass or lower, especially preferably 30% by mass or lower, particularly preferably 25% by mass or lower, specially preferably 20% by mass or lower, most preferably 15% by mass or lower, particularly most preferably 10% by mass or lower, exceptionally preferably 7% by mass or lower.

When the concentration of the protein particles is equal to or lower than the above-described upper limit, an increase in viscosity at the time of dispersing the protein particles in a medium is inhibited, so that the ease of handling tends to be improved. When the concentration of the protein particles is equal to or higher than the above-described lower limit, the interface between water and an oil-and-fat is sufficiently covered with the protein particles at the time of forming an emulsion composition; therefore, in a step that involves a change in the state of a continuous phase and/or a discontinuous phase of the emulsion composition, a sufficient emulsification stabilizing function (stabilization associated with adsorption of the protein particles to the interface between water and an oil-and-fat) tends to be provided.

A dispersion medium of the aqueous dispersion of the protein particles is usually water. The dispersion medium of the aqueous dispersion of the protein particles may also contain other components within a range that does not hinder the measurement of the protein particles, for example, in an amount of 0.5% by mass or less, preferably 0.1% by mass or less, more preferably 0.05% by mass or less, still more preferably 0.01% by mass or less.

Examples of the other components include lower alcohols, emulsifiers, oil-and-fats, salts, and ashes such as minerals.

Gel filtration chromatography of the aqueous dispersion of the protein particles is performed in the following manner.
(1) The aqueous dispersion containing the protein particles is centrifuged, and the resulting supernatant is recovered and analyzed.
(2) The supernatant obtained in (1) is filtered through a cartridge filter, and the thus obtained filtrate is identified by gel filtration chromatography (GPC) (see, for example, Japanese Unexamined Patent Application Publication No. H8-269075). The pore size of the cartridge filter is usually 1 µm or less, preferably 0.8 µm or less, more preferably 0.45 µm or less.

The filtrate may be directly subjected to the GPC measurement, or may be dried into a powder form using a centrifugal evaporator, re-dissolved or re-dispersed in an eluent for GPC measurement, and then promptly (within 3 hours) subjected to the GPC measurement. It is more preferred to employ a method in which the filtrate is dried into a powder form, re-dissolved or re-dispersed in an eluent for GPC measurement, and then promptly (within 3 hours) subjected to the GPC measurement.

It is preferred that the protein particles according to the present embodiment have at least one peak in a region of a molecular weight of 66,000 or more in terms of polyethylene glycol in a gel filtration chromatography elution curve.

It is noted here that a region of a specific molecular weight or more in terms of polyethylene glycol refers to a region in an elution curve of gel permeation chromatography, in which the elution time is equal to or shorter than the elution time obtained by subjecting a polyethylene glycol having the specific molecular weight to gel filtration chromatography under the same conditions. The number of peaks in the region of a specific molecular weight or more may be 2 or more, or 3 or more. An upper limit thereof is not particularly limited; however, it is usually 10 or less, preferably 5 or less, more preferably 1.

A lower limit of the molecular weight of the above-described peak is usually 66,000 or more, preferably 70,000 or more, more preferably 75,000 or more, still more preferably 80,000 or more, especially preferably 85,000 or more, particularly preferably 90,000 or more, specially preferably 95,000 or more, most preferably 100,000 or more, particularly most preferably 110,000 or more, exceptionally preferably 120,000 or more.

Meanwhile, an upper limit of the molecular weight of the above-described peak is usually 8,000,000 or less, preferably 7,500,000 or less, more preferably 7,000,000 or less, still more preferably 6,500,000 or less, especially preferably 6,000,000 or less, particularly preferably 5,000,000 or less, specially preferably 4,000,000 or less, most preferably 3,000,000 or less, particularly most preferably 2,000,000 or less, exceptionally preferably 1,550,000 or less.

The term "peak" used herein means a distinct peak in the elution curve at which the numerical value measured using a differential refractive index (RI) detector as a detection unit is maximum, and does not include a so-called "shoulder" where the sign of the slope of the elution curve does not change.

By using protein particles exhibiting the above-described molecular weight distribution in an emulsion composition, aggregation and coalescence of the inner phase of the emulsion composition are inhibited when the inner phase and/or the outer phase of the emulsion composition undergo a change in state. As a result, a change in the size of the inner phase of the emulsion composition is inhibited, so that the stability of the emulsion composition can be improved. Further, by incorporating protein particles exhibiting the above-described molecular weight distribution into an emulsion composition, the stability of the emulsion composition during long-term storage can be improved, which is more preferred.

Moreover, when the molecular weight is in the above-described range, appropriate viscosity and size are obtained when the protein particles are dispersed in a medium, so that a good texture can be obtained.

Protein particles satisfying the above-described condition (A) can be obtained by adjusting the conditions of the below-described heat treatment and/or pressurization treatment.

The protein particles according to the present embodiment have a weight-average molecular weight in terms of polyethylene glycol, which is measured by gel filtration chromatography, of usually more than 27,000, preferably 30,000 or more, more preferably 35,000 or more, still more preferably 40,000 or more, especially preferably 45,000 or more, particularly preferably 50,000 or more, specially preferably 55,000 or more, most preferably 60,000 or more, particularly most preferably 65,000 or more, exceptionally preferably 70,000 or more.

An upper limit of the weight-average molecular weight is usually 1,500,000 or less, preferably 1,250,000 or less, more preferably 1,000,000 or less, still more preferably 800,000 or less, especially preferably 600,000 or less, particularly preferably 500,000 or less, specially preferably 400,000 or less, most preferably 300,000 or less, particularly most preferably 200,000 or less, exceptionally preferably 150,000 or less.

When the molecular weight is equal to or less than the above-described upper limit, an increase in viscosity at the time of dispersing the protein particles in a medium is inhibited, so that the ease of handling tends to be improved. When the molecular weight of the protein particles is equal to or more than the above-described lower limit, a sufficient emulsification stabilizing function (stabilization associated with adsorption of the protein particles to the interface between water and an oil-and-fat) tends to be provided in a step that involves a change in the state of a continuous phase and/or a discontinuous phase of an emulsion composition.

Protein particles satisfying the above-described condition (B) can be obtained by adjusting the conditions of the below-described heat treatment and/or pressurization treatment.

The protein particles according to the present embodiment have a number-average molecular weight in terms of polyethylene glycol, which is measured by gel filtration chromatography, of usually more than 3,500, preferably 3,600 or more, more preferably 3,800 or more, still more preferably 4,000 or more, especially preferably 4,200 or more, particularly preferably 4,400 or more, specially preferably 4,600 or more, most preferably 4,800 or more, particularly most preferably 5,000 or more, exceptionally preferably 5,200 or more.

An upper limit of the number-average molecular weight is usually 1,500,000 or less, preferably 1,000,000 or less, more preferably 500,000 or less, still more preferably 100,000 or less, especially preferably 50,000 or less, particularly preferably 40,000 or less, specially preferably 20,000 or less, most preferably 10,000 or less, particularly most preferably 8,000 or less, exceptionally preferably 6,000 or less.

When the molecular weight is equal to or less than the above-described upper limit, an increase in viscosity at the time of dispersing the protein particles in a medium is inhibited, so that the ease of handling tends to be improved. When the molecular weight of the protein particles is equal to or more than the above-described lower limit, a sufficient emulsification stabilizing function (stabilization associated with adsorption of the protein particles to the interface between water and an oil-and-fat) tends to be provided in a step that involves a change in the state of a continuous phase and/or a discontinuous phase of an emulsion composition.

Protein particles satisfying the above-described condition (C) can be obtained by adjusting the conditions of the below-described heat treatment and/or pressurization treatment.

The peak molecular weight (Mp), the weight-average molecular weight, and the number-average molecular weight, all of which are in terms of polyethylene glycol, are determined based on a relational expression obtained by subjecting polyethylene glycols of known molecular weight to gel filtration chromatography under the same conditions and performing third-order approximation of the relationship between the molecular weight and the elution time. The number of polyethylene glycols used for obtaining the relational expression is usually 2 or more, preferably 3 or more, more preferably 5 or more, and the larger the number, the more preferred it is. An upper limit thereof is not particularly limited and may be 50 or less. For example, the number of polyethylene glycols used for obtaining the relational expression may be 12.

Further, it is preferred that the elution times of the peaks in the elution curve of the protein particles be all included between a maximum value and a minimum value of the elution times of the polyethylene glycols used for obtaining the relational expression.

The shape of the protein particles according to the present embodiment is not particularly limited, and examples thereof include a spherical shape, a rod-like shape, a stringlike shape, a gel-like shape, a web-like shape, a porous shape, a needle-like shape, a flaky shape, and a bar-like shape. When the protein particles are in the form of a gel, the gel may be shrunk or swollen.

In a dispersion medium, the protein particles according to the present embodiment have an average particle size of usually 100 µm or less, preferably 50 µm or less, more preferably 20 µm or less, still more preferably 10 µm or less, especially preferably 5 µm or less, particularly preferably 1 µm or less, specially preferably 0.8 µm or less, most preferably 0.5 µm or less, particularly most preferably 0.3 µm or less, exceptionally preferably 0.2 µm or less.

A lower limit of the average particle size is usually 0.001 µm or more, preferably 0.003 µm or more, more preferably 0.005 µm or more, still more preferably 0.008 µm or more, especially preferably 0.01 µm or more, particularly preferably 0.02 µm or more, specially preferably 0.03 µm or more, most preferably 0.04 µm or more, particularly most preferably 0.05 µm or more, exceptionally preferably 0.06 µm or more.

When the average particle size of the protein particles in a dispersion is in the above-described range, the protein particles moderately act on the taste receptors on the tongue surface without deteriorating the texture at the time of eating (the protein particles give moderate smoothness down the throat without causing the feeling of roughness on the tongue), allowing the taste such as umami to be perceived moderately.

The average particle size can be measured in a batchwise manner using the protein particles diluted with demineralized water as appropriate, or a "dispersion obtained by removing oil droplets and/or oil-and-fat from an emulsion composition", and a laser diffraction/scattering-type particle size distribution analyzer LA-950 (manufactured by Horiba, Ltd.). The analysis conditions are not limited; however, analysis is preferably performed based on number.

In cases where it is difficult to perform the measurement using a laser diffraction/scattering-type particle size distribution analyzer due to insufficient intensity of diffracted/scattered light or the like, a dynamic light scattering method can be employed to measure the particle size distribution, the average particle size, and the median diameter of the protein particles in a state of being dispersed in a liquid. The measurement results obtained by the dynamic light scattering method can be analyzed by, for example, a cumulant method.

The protein particles according to the present embodiment are preferably hardly soluble in aqueous-phase components and oil-phase components, more preferably insoluble in aqueous-phase components and oil-phase components. The protein particles prior to being dispersed in an aqueous phase or an oil phase may be in a powder form, a paste form, or a pellet form.

A raw material protein constituting the protein particles, or the protein particles have an isoelectric point of usually a pH of 2 or higher, preferably a pH of 2.6 or higher, more preferably a pH of 2.8 or higher, still more preferably a pH of 3 or higher, especially preferably a pH of 3.2 or higher, particularly preferably a pH of 3.4 or higher, specially preferably a pH of 3.6 or higher, most preferably a pH of 3.8 or higher, particularly most preferably a pH of 4 or higher, and exceptionally preferably a pH of 4.1 or higher.

An upper limit value of the isoelectric point is usually a pH of 12 or lower, preferably a pH of 11 or lower, more preferably a pH of 10 or lower, still more preferably a pH of 9 or lower, especially preferably a pH of 8 or lower, particularly preferably a pH of 7.5 or lower, specially preferably a pH of 7 or lower, most preferably a pH of 6.5 or lower, particularly most preferably a pH of 6 or lower, and exceptionally preferably a pH of 5.8 or lower.

When the isoelectric point is in the above-described range, since the protein itself has a moderate affinity for water or oil-and-fat, the wettability can be controlled at a preferred level.

Examples of the raw material protein constituting the protein particles include animal proteins, plant proteins, fungus-derived proteins, and proteins that are artificially produced based on genetic information.

Thereamong, plant proteins and fungus-derived proteins are preferred since these proteins can address the problem of allergies to milk-derived components. Plant proteins are preferred also from the standpoint of providing a food conforming to religious restrictions and vegetarianism.

The raw material protein may be a purified product prepared by extraction, separation, and/or concentration of a protein from an animal, plant, or fungal raw material.

The raw material protein may be used singly, or in combination of two or more kinds thereof.

Examples of the plant proteins include Fabaceae plant-derived proteins, Gramineae plant-derived proteins, Chenopodiaceae plant-derived proteins, and Brassicaceae plant-derived proteins.

Examples of the Fabaceae plant include *Phaseolus* plants, *Cicer* plants, *Pisum* plants, *Lens* plants, and *Lupinus* plants.

Examples of the Gramineae plant include wheat (*Triticum* L.), oat (common wild oat; *Avena fatua* L.), rye (*Secale cereale* L.), barley (*Hordeum vulgare* L.), corn (*Zea mays L*.), great millet (*Sorghum bicolor* (L.) Moench), and rice (*Oryza sativa*)*.*

Examples of the Chenopodiaceae plant include beet (*Beta vulgaris*), sugar beet (*Beta vulgaris* ssp. *vulgaris* var. *altissima*), and spinach (*Spinacia oleracea* L.).

Examples of the Brassicaceae plant include cabbage (*Brassica oleracea* var. *capitata*) and brassica rapa (*Brassica campestris L.).*

Among the above-exemplified plant proteins, Fabaceae plant-derived proteins are preferred since Fabaceae plants are richer in protein content than other plants.

Fabaceae plant-derived proteins are also preferred from the standpoint of reducing the global environmental load in the production.

Fabaceae plants can utilize nitrogen in the air by symbiosis with rhizobia, and are thus capable of growing even in a poor soil that lacks nitrogen nutrients. Therefore, it is expected that Fabaceae plants can be cultivated independent of a nitrogen fertilizer.

Among Fabaceae plants, *Pisum* plants are preferred since they are less allergenic than soybeans and have better digestibility.

Examples of the *Pisum* plants include yellow pea (*Pisum Sativum*), green pea, purple pea, blue pea, red pea, and white pea.

In the present specification, the protein particles may also contain components other than the protein.

The content of the protein in the protein particles is usually 5% by mass or more, preferably 10% by mass or more, more preferably 30% by mass or more, still more preferably 50% by mass or more, especially preferably 80% by mass or more, particularly preferably 90% by mass or more, specially preferably 95% by mass or more, and most preferably 100% by mass.

Examples of the components other than the protein include water, lipids, and minerals. Particularly, these components may be contained as impurities when the protein is a purified product prepared from an animal, plant, or fungal raw material.

### (Method of Producing Protein Particles)

Protein particles can be produced by, for example, a production method that includes: a step of mixing water and a raw material protein to prepare a mixture; and a step of heat-treating the mixture at 60°C to 200°C for 1 second to 120 minutes.

By adjusting the conditions of the heat treatment, protein particles satisfying the above-described conditions (A) to (C) can be obtained. In addition, not only an appropriate size is obtained through simple binding of protein molecules, but also the physical properties of the resulting protein particles, such as "wettability that allows stabilization of an interface between water and an oil-and-fat", "size", "relaxation ability of particles to withstand a change in the state of a liquid phase", are adjusted appropriately.

The temperature during the heat treatment is usually 60°C or higher, preferably 65°C or higher, more preferably 70°C or higher, still more preferably 80°C or higher, especially preferably 90°C or higher, particularly preferably 95°C or higher, specially preferably 100°C or higher, most preferably 105°C or higher, particularly most preferably 110°C or higher, exceptionally preferably 120°C or higher.

An upper limit temperature during the heat treatment may be any temperature as long as it is not higher than the decomposition temperature of the raw material protein, and it is usually 200°C or lower, preferably 190°C or lower, more preferably 180°C or lower, still more preferably 170°C or lower, especially preferably 160°C or lower, particularly preferably 155°C or lower, specially preferably 150°C or lower, most preferably 145°C or lower, particularly most preferably 140°C or lower, exceptionally preferably 130°C or lower.

When the temperature during the heat treatment is in the above-described range, desired physical properties of the protein particles are obtained efficiently; therefore, an emulsification stabilizing effect exerted by the protein particles can be obtained.

When the temperature during the heat treatment is excessively low, a conformational change of protein is unlikely to occur, and the resulting protein particles do not achieve desired physical properties as a result; therefore, it tends to be difficult to efficiently obtain an emulsification stabilizing effect exerted by the protein particles.

When the temperature during the heat treatment is excessively low, excessive denaturation and decomposition of protein tend to make it difficult to efficiently obtain desired physical properties of the protein particles and to obtain an emulsification stabilizing effect exerted by the protein particles.

The time of the heat treatment is usually 1 second or longer, preferably 2 seconds or longer, more preferably 5 seconds or longer, still more preferably 10 seconds or longer, especially preferably 1 minute or longer, particularly preferably 5 minutes or longer, specially preferably 15 minutes or longer, most preferably 30 minutes or longer.

An upper limit of the time of the heat treatment is usually 120 minutes or shorter, preferably 115 minutes or shorter, more preferably 110 minutes or shorter, still more preferably 105 minutes or shorter, especially preferably 100 minutes or shorter, particularly preferably 95 minutes or shorter, specially preferably 90 minutes or shorter, most preferably 85 minutes or shorter, particularly most preferably 80 minutes or shorter, exceptionally preferably 70 minutes or shorter.

An excessively long or excessively short treatment time tends to make it difficult to efficiently obtain desired physical properties.

The method of producing protein particles may include the step of performing a treatment under a pressure other than normal pressure, in addition to the above-described heat treatment step. The treatment under a pressure other than normal pressure may be performed after the heat treatment, or the step of performing the treatment under a pressure other than normal pressure may be performed simultaneously with the heat treatment. It is more preferred to perform the heat treatment under a pressure other than normal pressure since this enables to shorten the process and efficiently produce protein particles.

By performing the heat treatment under a pressure other than normal pressure, desired physical properties of the protein particles are obtained efficiently; therefore, an emulsification stabilizing effect exerted by the protein particles can be obtained. By combining heating and pressurization, the heating temperature can be lowered and the process time can be shortened, so that protein particles can be produced efficiently.

In the case of performing a treatment under a pressure other than normal pressure, the gauge pressure is usually a value larger than 0 MPa or smaller than 0 MPa.

Particularly, a lower limit of the gauge pressure is preferably higher than 0 MPa, more preferably 0.02 MPa or higher, still more preferably 0.05 MPa or higher, especially preferably 0.06 MPa or higher, particularly preferably 0.07 MPa or higher, specially preferably 0.08 MPa or higher, most preferably 0.09 MPa or higher, particularly most preferably 0.10 MPa or higher, and exceptionally preferably 0.11 MPa or higher.

An upper limit of the gauge pressure is preferably 800 MPa or lower, more preferably 500 MPa or lower, still more preferably 300 MPa or lower, especially preferably 250 MPa or lower, particularly preferably 150 MPa or lower, specially preferably 50 MPa or lower, most preferably 10 MPa or lower, particularly most preferably 1 MPa or lower, and exceptionally preferably 0.50 MPa or lower.

By performing the treatment under a pressure in the above-described range, desired physical properties of the protein particles are obtained efficiently; therefore, an emulsification stabilizing effect exerted by the protein particles can be obtained.

The method for producing protein particles may also include the stirring step in combination with the heating and/or pressurization treatment step.

Further, the method for producing protein particles may include a step of performing a physical treatment by UV irradiation or the like, and/or a chemical treatment with an acid, an alkali, a denaturant, or the like, in addition to the heating and/or pressurization treatment step. By performing such treatment(s), the protein can be physically and/or chemically modified (e.g., denatured), whereby the physical properties, i.e., the molecular weight and/or the wettability, of the resulting protein particles can be controlled.

In other words, the protein controlled to have appropriate physical properties is more likely to exist in the interface to be stabilized (interface between water and the oil-and-fat), so that a stable emulsion composition can be formed. Further, by performing the heating and/or pressurization treatment and the treatment by UV irradiation or the like, a sterilization effect which prevents the decomposition of materials themselves is expected to be obtained as well.

These treatments may be performed singly, or two or more thereof that are selected as desired may be performed simultaneously or separately.

For example, a denaturant is added to a solution containing the protein, and a heat is applied to the resultant. By this, a denaturation treatment with the denaturant and a denaturation treatment with the heat are performed simultaneously. A method of these denaturation treatments may be selected taking into consideration the type of the protein to be denatured, the extent of required denaturation, and the like.

For example, in the case of performing a heat treatment, the heat treatment may be dry heating or wet heating. For the dry heating, a roasting apparatus, a hot-air heating apparatus, or a microwave heating apparatus can be used. For the wet heating, a humidifying/heating apparatus, a steaming apparatus, or a steam-heating apparatus can be used.

In order to obtain protein particles having desired physical properties, intermolecular crosslinking or molecular modification may be performed. Examples of a crosslinking agent used for the crosslinking or a modifier used for the modification include organic compounds such as glutaraldehyde, and naturally-occurring substances. From the standpoint of reducing the environmental load and preventing health hazards, it is preferred to use a naturally-occurring substance as the crosslinking agent or the modifier. Alternatively, intermolecular crosslinking or molecular modification may be performed by a step of heating and/or pressurizing only the raw material protein without using a crosslinking agent or a modifier. In this case, there is an advantage that the raw material cost is reduced and clean labeling is achieved.

From the viewpoint of controlling the dispersibility and the particle size of the protein particles of the present embodiment, the protein particles may be separately subjected to a crushing treatment, a pulverization treatment, and/or a dispersion treatment. Methods of these treatments are not limited, and each treatment may be performed by a dry method or a wet method. A combination of these methods may be employed to perform the crushing treatment, the pulverization treatment, and/or the dispersion treatment in a stepwise manner.

Examples of a wet treatment method include the use of a high-pressure homogenizer, a homogenizer, a jet mill, a vibration mill, a tumbling mill, a high-pressure fluid impact mill, a paint shaker, a bead mill, a ball mill, a disk mill, a homomixer, or the like. Examples of a dry treatment method include the use of a pin mill, a jet mill, a ball mill, a hammer mill, a roller mill, a cutter mill, an impact shearing mill, or the like.

Thereamong, a high-pressure homogenizer, a bead mill, a cutter mill, or a hammer mill is preferably used.

The temperature during the crushing treatment, the pulverization treatment, or the dispersion treatment is usually -196°C or higher, preferably -150°C or higher, more preferably -100°C or higher, still more preferably -80°C or higher, especially preferably -40°C or higher, particularly preferably -20°C or higher, specially preferably 0°C or higher, most preferably 10°C or higher, particularly most preferably 20°C or higher, and exceptionally preferably room temperature (25°C) or higher.

An upper limit temperature during the treatment is usually 100°C or lower, preferably 98°C or lower, more preferably 95°C or lower, still more preferably 93°C or lower, especially preferably 90°C or lower, particularly preferably 88°C or lower, specially preferably 85°C or lower, most preferably 80°C or lower, particularly most preferably 78°C or lower, and exceptionally preferably 75°C or lower.

The treatment time is usually 1 second or longer, preferably 2 seconds or longer, more preferably 5 seconds or longer, still more preferably 10 seconds or longer, especially preferably 20 seconds or longer, particularly preferably 30 seconds or longer, specially preferably 1 minute or longer, most preferably 1.5 minutes or longer, particularly most preferably 2 minutes or longer, and exceptionally preferably 3 minutes or longer.

Meanwhile, the treatment time is usually 10 hours or shorter, preferably 8 hours or shorter, more preferably 7 hours or shorter, still more preferably 6 hours or shorter, especially preferably 5 hours or shorter, particularly preferably 3 hours or shorter, specially preferably 2 hours or shorter, most preferably 1 hour or shorter, particularly most preferably 30 minutes or shorter, and exceptionally preferably 10 minutes or shorter.

An excessively short treatment time tends to make it difficult to control the particle size, while an excessively long treatment time tends to deteriorate the productivity.

For the production of the protein particles of the present embodiment, crushed particles obtained by the above-described production method may be subjected to a classification treatment based on the particle size.

As for the conditions of the classification treatment, the mesh size is usually 150 µm or less, preferably 106 µm or less, more preferably 53 µm or less, still more preferably 45 µm or less, especially preferably 38 µm or less, particularly preferably 20 µm or less.

An apparatus used for the classification treatment is not particularly limited.

In the case of dry sieving, for example, a trommel sieve, a shaking sieve, a rotary sieve, or a vibrating sieve may be used.

In the case of dry airflow classification, for example, a gravity classification apparatus, an inertial classification apparatus, or a centrifugal classification apparatus (e.g., a classifier or a cyclone) may be used.

In the case of wet classification, for example, a mechanical wet classification apparatus, a hydraulic classification apparatus, a sedimentation classification apparatus, or a centrifugal wet classification apparatus may be used.

<Protein>

Next, a second embodiment of the present invention will be described.

The second embodiment of the present invention is a protein that satisfies one of the following conditions (A) to (C) and is partially in the state of particles when made into an aqueous dispersion:
(A): having at least one peak in a region of a molecular weight of 66,000 or more in terms of polyethylene glycol in a gel filtration chromatography elution curve;
(B): having a weight-average molecular weight of more than 27,000 in terms of polyethylene glycol as measured by gel filtration chromatography; and
(C): having a number-average molecular weight of more than 3,500 in terms of polyethylene glycol as measured by gel filtration chromatography.

The protein preferably satisfies at least two selected from the conditions (A) to (C), more preferably satisfies all of the three conditions (A) to (C).

Although a detailed mechanism is not clear, when a protein satisfying at least one of the above-described conditions is incorporated into an emulsion composition, since the protein has appropriate physical properties in terms of "wettability that allows stabilization of an interface between water and an oil-and-fat", "size", "relaxation ability of particles to withstand a change in the state of a liquid phase", and the like, an emulsion composition that maintains its quality even when stored frozen can be obtained.

The protein according to the present embodiment is obtained not only by binding of a raw material protein, which gives an appropriate size, but also by crosslinking, entanglement, and the like between protein molecules.

Therefore, in the protein according to the present embodiment, a region (region 1) that is strongly affected by crosslinking and entanglement of protein molecules turns into the state of particles when the protein is made into an aqueous dispersion.

In the protein, some of the particles and a region (region 2) that is not in the state of particles exhibit a unique molecular weight due to binding of the raw material protein. In the below-described gel filtration chromatography measurement, the molecular weight of this region 2 is measured.

It is noted here that the term "state of particles" used herein means a state of containing a particle component (granulated substaces) that is not dissolved in water, namely protein particles.

A method of measuring the protein by gel filtration chromatography is the same as the method described above in the section of "Protein Particles".

Preferred ranges and preferred reasons of the conditions (A) to (C) are also the same as those described above in the section of "Protein Particles".

The protein according to the present embodiment is partially in the state of particles when made into an aqueous dispersion. The particles, which are particles of the protein, are the same as the particles described above in the section of "Protein Particles".

The shape and the average particle size of the particles of the protein are also the same as those described above in the section of "Protein Particles".

The particles of the protein are preferably hardly soluble in aqueous-phase components and oil-phase components, more preferably insoluble in aqueous-phase components and oil-phase components.

The protein prior to being dispersed in an aqueous phase or an oil phase may be in a powder form, a paste form, or a pellet form.

The raw material protein constituting the protein according to the present embodiment is preferably a plant protein.

The plant protein is preferably a protein derived from a Fabaceae plant.

The Fabaceae plant is preferably a *Phaseolus, Cicer, Pisum, Lens,* or *Lupinus* plant.

The raw material protein is the same as the raw material protein described above in the section of "Protein Particles".

### (Method of Producing Protein)

A method of producing the protein according to the present embodiment includes the following steps (1) and (2):
Step (1): a step of mixing water and a raw material protein to prepare a mixture; and
Step (2): a step of heat-treating the mixture at 60°C to 200°C for 1 second to 120 minutes.

The step (1) is the step of mixing water and a raw material protein to prepare a mixture, and the raw material protein is as described above.

A method of mixing water and the raw material protein is not particularly limited, and any known mixing method may be employed.

The step (2) is the step of heat-treating the mixture obtained in the step (1), at 60°C to 200°C for 1 second to 120 minutes.

The temperature and the time of the heat treatment are the same as those described above in the section of "Method of Producing Protein Particles".

Another embodiment of the present invention is a method of producing a protein, which method includes the following steps (1) and (2') and which protein is partially in the state of particles when made into an aqueous dispersion:
Step (1): a step of mixing water and a raw material protein to prepare a mixture; and
Step (2'): a step of heat-treating the mixture at 60°C to 200°C for 1 second to 120 minutes under a pressure other than normal pressure.

In this method, the raw material protein is a plant protein.

The step (1) is the step of mixing water and a raw material protein to prepare a mixture, and the raw material protein is a plant protein. The plant protein is as described above.

A method of mixing water and the raw material protein is not particularly limited, and any known mixing method may be employed.

The step (2') is the step of heat-treating the mixture obtained in the step (1), at 60°C to 200°C for 1 second to 120 minutes under a pressure other than normal pressure.

The temperature, the time, and the pressure of the heat treatment are the same as those described above in the section of "Method of Producing Protein Particles".

### <Protein Particle Dispersion>

When the protein according to the second embodiment of the present invention is mixed with a medium (dispersion medium), a dispersion containing a particle component not dissolved in the medium is obtained. This dispersion is hereinafter referred to as "protein particle dispersion". The size and the form of protein particles in the medium are the same as those described above in the section of "Protein Particles". The protein preferably contains a component that is hardly soluble in the medium, more preferably contains a component that is insoluble in the medium.

Examples of the main component constituting the dispersion medium include water, oils, and fats. The term "main component" used herein refers to, among constituents, one which has the highest content ratio. The main component constituting the dispersion medium of the protein particles is preferably water. When the protein particles exist in the medium containing water as its main component, since the dispersion state is appropriate, the protein particles are unlikely to deteriorate the texture in eating. Further, since the dispersion state is appropriate, an emulsification stabilizing effect exerted by the protein particles can be obtained in the formation of the below-described emulsion composition.

The pH of the protein particle dispersion is not particularly limited, and a suitable pH may be selected in accordance with the intended use. For example, when the protein particle dispersion is used in a food application, the protein particle dispersion may have any pH as long as it is edible and drinkable; however, a lower limit value of the pH is usually higher than 1, preferably 2 or higher, more preferably 3 or higher, still more preferably 4 or higher, especially preferably 5 or higher, particularly preferably 5.5 or higher, specially preferably 6 or higher, most preferably 6.2 or higher, particularly most preferably 6.3 or higher, and exceptionally preferably 6.5 or higher.

An upper limit value of the pH is usually 13 or lower, preferably 12 or lower, more preferably 10 or lower, still more preferably 9.5 or lower, especially preferably 9 or lower, particularly preferably 8.5 or lower, specially preferably 8 or lower, most preferably 7.8 or lower, particularly most preferably 7.5 or lower, and exceptionally preferably 7.4 or lower.

The pH of the protein particle dispersion may be around the isoelectric point of the protein particles, the protein, or the raw material protein constituting the protein particles or the protein; however, from the standpoint of avoiding the generation of coarse aggregates and precipitates, the pH of the protein particle dispersion is usually apart from the isoelectric point by at least 0.5, preferably by at least 1.

By controlling the pH to be in this range, the protein particles and/or the protein can be prevented from aggregating and can thus maintain a preferred size, so that a good texture in eating can be maintained.

### <Emulsion Composition>

The emulsion composition according to yet another embodiment of the present invention is an emulsion composition which contains water, an oil-and-fat, and protein particles, and is characterized in that: the protein particles exist in an interface between the water and the oil-and-fat; and the protein particles are the protein particles according to the first embodiment of the present invention.

Alternatively, the emulsion composition according to the present embodiment may be an emulsion composition which contains water, an oil-and-fat, and a protein, and is characterized in that: the protein exists in an interface between the water and the oil-and-fat; and the protein is the protein according to the second embodiment of the present invention.

The emulsion composition according to the present embodiment may be an oil-in-water (O/W-type) emulsion composition or a water-in-oil (W/O-type) emulsion composition, preferably an oil-in-water emulsion composition. The oil-in-water emulsion composition encompasses a multi-phase emulsion composition, such as a W/O/W-type emulsion composition.

The emulsion composition according to the present embodiment is a composition emulsified by protein particles or a protein. In this case, the term "emulsified" can be paraphrased as "a state in which the protein particles or the protein exist in an interface between water and an oil-and-fat". The emulsion composition according to the present embodiment is emulsified by the protein particles according to the first embodiment of the present invention or the protein according to the second embodiment of the present invention; therefore, the emulsion composition is characterized by having high emulsion stability (heat resistance, cooling resistance, freezing resistance, thawing resistance, and long-term stability) that withstands changes in state (e.g., a change from a liquid state to a crystallized or solid state, and a change from a crystallized or solid state to a liquid state) of a discontinuous phase constituting an inner phase and/or a continuous phase constituting an outer phase.

In the above-described emulsion composition, the protein particles or the protein usually exist in an interface between water and the oil-and-fat; however, the emulsion composition is not limited to this mode, and may contain the protein particles or the protein that are dispersed in water or the oil-and-fat.

### (Water)

The water contained in the emulsion composition according to the present embodiment may contain a lower alcohol, a polyhydric alcohol, or the like.

The content of water in the emulsion composition is usually 20% by mass or more, preferably 30% by mass or more, more preferably 35% by mass or more, still more preferably 40% by mass or more, especially preferably 45% by mass or more, particularly preferably 50% by mass or more, specially preferably 55% by mass or more, most preferably 60% by mass or more, particularly most preferably 70% by mass or more, and exceptionally preferably 80% by mass or more, with respect to a total amount of the emulsion composition.

Meanwhile, the content of water in the emulsion composition is usually less than 100% by mass, preferably 99% by mass or less, more preferably 98% by mass or less, still more preferably 97% by mass or less, especially preferably 96% by mass or less, particularly preferably 95% by mass or less, specially preferably 94% by mass or less, most preferably 93% by mass or less, particularly most preferably 92% by mass or less, and exceptionally preferably 91% by mass or less.

When the content of water in the emulsion composition is in the above-described range, a stable emulsion composition can be easily obtained, which is an effect of the present application.

### (Oil-and-Fat)

Examples of the oil-and-fat contained in the emulsion composition of the present embodiment include unsaturated higher fatty acid hydrocarbons, unsaturated higher fatty acids, animal/plant-derived oils and fats, isoprenoids including squalene and tocopherol, higher alcohols, synthetic ester oils, glycol higher fatty acid esters, saturated fatty acids, and unsaturated fatty acids.

The oil-and-fat preferably contains a component that can be used for a food (hereinafter referred to as "edible oil-and-fat"), and any edible oil-and-fat may be used. The "edible oil-and-fat" encompasses: oils and fats having physiological functions; fat-soluble pigments; and antioxidants.

Examples of the edible oil-and-fat include: vegetable oils and fats, such as rapeseed oil, rice oil, soybean oil, corn oil, safflower oil, sunflower oil, cottonseed oil, sesame oil, olive oil, palm oil, palm kernel oil, coconut oil, linseed oil, macadamia nut oil, camellia seed oil, tea seed oil, rice bran oil, and cocoa butter; animal oils and fats, such as milk fat, beef tallow, lard, chicken fat, mutton tallow, and fish oil; hydrogenated or processed oils and fats that are produced from liquid or solid products of the above-described vegetable or animal oils and fats through oil-and-fat processing by purification, deodorization, fractionation, hydrogenation, or transesterification, such as hydrogenated coconut oil and hydrogenated palm kernel oil; and liquid oils and solid fats that are obtained by further fractionation of the above-described oils and fats.

In addition, an oil or fat having physiological functions can be used, and examples thereof include docosahexaenoic acid (DHA), eicosapentaenoic acid (EPA), arachidonic acid, *α-*linolenic acid, γ-linolenic acid, and medium-chain fatty acid triglyceride (MCT).

These oils and fats may be used singly, or in combination of two or more kinds thereof.

Further, a fat-soluble pigment and an antioxidant can be used.

Examples of the pigment include: carotenoid pigments, such as annatto pigment, *β*-carotene, paprika pigment, carrot carotene, and *Dunaliella* carotene; *Monascus* pigment; chlorophylls; turmeric pigments such as curcumin (curcuminoid); and edible tar pigments.

Examples of the antioxidant include: plant extracts, such as rosemary extracts, tea extracts, raw coffee bean extracts, grape seed extracts, and myrica extracts; tocopherol; tocotrienol; ascorbyl palmitate; dibutylhydroxytoluene; and butylhydroxyanisole.

From the standpoint of taste, the oil-and-fat is particularly preferably a fat that is solid at normal temperature. The fat that is solid at normal temperature is an oil-and-fat that exists in a solid state at normal temperature (25°C), and examples thereof include beef tallow, lard, palm stearin, palm medium-melting-point fraction, hydrogenated coconut oil, hydrogenated palm kernel oil, hydrogenated rapeseed oil, hydrogenated castor oil, hydrogenated soybean oil, hydrogenated beef tallow oil, and hydrogenated fish oil.

A vegetable oil-and-fat, or a hydrogenated or processed oil-and-fat thereof is more preferably used.

More preferred examples of the oil-and-fat include: hydrogenated vegetable or animal oils and fats, such as palm oil, palm stearin, palm kernel oil, coconut oil, cocoa butter, milk fat, beef tallow, lard, chicken fat, mutton tallow, hydrogenated coconut oil, and hydrogenated palm kernel oil; solid fats obtained by fractionation of a vegetable or animal oil or fat that has been hydrogenated or processed; and medium-chain fatty acid triglycerides (MCT).

Still more preferred examples of the oil-and-fat include palm kernel oil, coconut oil, milk fat, hydrogenated coconut oil, hydrogenated palm kernel oil, and medium-chain fatty acid triglycerides (MCT).

Especially preferred examples of the oil-and-fat include palm kernel oil, coconut oil, hydrogenated coconut oil, and hydrogenated palm kernel oil.

These oils and fats may be used singly, or in combination of two or more kinds thereof.

Particularly, regarding the edible oil-and-fat, from the standpoint of obtaining a higher quality of taste, the ratio of fatty acids other than saturated fatty acids, i.e., the ratio of unsaturated fatty acids including trans fatty acids, with respect to all fatty acids bound to triglyceride molecules contained as a main component is preferably 50% by mass or less, more preferably 30% by mass or less, still more preferably 20% by mass or less, especially preferably 10% by mass or less, and particularly preferably 5% by mass or less.

Further, in the edible oil-and-fat, the ratio of fatty acids having 12 or less carbon atoms with respect to all fatty acids bound to the triglyceride molecules is preferably 1% by mass or more, more preferably 3% by mass or more, still more preferably 5% by mass or more, especially preferably 10% by mass or more, and particularly preferably 30% by mass or more.

From the standpoint of obtaining a good flavor without an oxidized odor in heating, the edible oil-and-fat has an iodine value of usually 60.0 or less, preferably 50.0 or less, more preferably 30.0 or less, still more preferably 20.0 or less, especially preferably 10.0 or less, and particularly preferably 5.0 or less.

Moreover, from the standpoint of producing a composition having a good flavor, the edible oil-and-fat has a solid fat content (SFC) at 10°C of usually 0% by mass or more, preferably 20% by mass or more, more preferably 30% by mass or more, still more preferably 40% by mass or more, and especially preferably 50% by mass or more.

It is noted here that the solid fat content (SFC) is generally measured by a method based on ordinary pulse NMR technique, and the use of solid fat index (SFI) determined by thermal analysis leads to little difference.

From the standpoint of producing a composition having a good flavor, the edible oil-and-fat has a slip melting point of usually -20°C or higher, preferably -15°C or higher, more preferably -10°C or higher, still more preferably 0°C or higher, especially preferably 5°C or higher, particularly preferably 10°C or higher, specially preferably 15°C or higher, most preferably 20°C or higher, particularly most preferably 25°C or higher, and exceptionally preferably 26°C or higher.

From the standpoint of obtaining a good emulsion stability, an upper limit of the slip melting point is usually 190°C or lower, preferably 100°C or lower, more preferably 90°C or lower, still more preferably 80°C or lower, especially preferably 70°C or lower, particularly preferably 60°C or lower, specially preferably 50°C or lower, most preferably 45°C or lower, particularly most preferably 40°C or lower, and exceptionally preferably 35°C or lower. The slip melting point can be measured by any known method.

The melting point can also be measured using DSC. The melting point of the edible oil-and-fat to be used is not limited; however, from the standpoint of producing a composition having a good flavor, the melting point is usually -20°C or higher, preferably -15°C or higher, more preferably - 10°C or higher, still more preferably 0°C or higher, especially preferably 5°C or higher, particularly preferably 10°C or higher, specially preferably 15°C or higher, most preferably 20°C or higher, particularly most preferably 25°C or higher, and exceptionally preferably 26°C or higher.

From the standpoint of obtaining a good emulsion stability, an upper limit of the melting point is usually 190°C or lower, preferably 100°C or lower, more preferably 90°C or lower, still more preferably 80°C or lower, especially preferably 70°C or lower, particularly preferably 60°C or lower, specially preferably 50°C or lower, most preferably 45°C or lower, particularly most preferably 45°C or lower, and exceptionally preferably 35°C or lower.

When the physical properties of the edible oil-and-fat are in the above-described respective ranges, good texture, appearance, tactile feel, viscosity, stability, and the like can be obtained when the edible oil-and-fat is made into an emulsion composition.

Further, in the present embodiment, an oil phase formed of the above-described oil-and-fat preferably has an average particle size of 1 µm or more. The average particle size of the oil phase refers to the size of the discontinuous phase of the above-described emulsion composition, i.e., the diameter of the oil phase in the O/W composition or W/O/W composition. By controlling the average particle size of the oil phase to be in the above-described range, good texture, appearance, tactile feel, viscosity, stability, and the like can be obtained.

The average particle size of the oil phase is usually more than 0.5 µm, preferably 1 µm or more, more preferably 1.2 µm or more, still more preferably 1.5 µm or more, especially preferably 2 µm or more, particularly preferably 2.2 µm or more, specially preferably 2.5 µm or more, most preferably 3 µm or more, particularly most preferably 3.5 µm or more, and exceptionally preferably 4 µm or more.

An upper limit of the average particle size is not limited; however, it is usually 1,000 µm or less, preferably 500 µm or less, more preferably 250 µm or less, still more preferably 100 µm or less, especially preferably 50 µm or less, particularly preferably 40 µm or less, specially preferably 30 µm or less, and most preferably 20 µm or less.

By controlling the average particle size of the oil phase to be in the above-described range, appropriate texture, appearance, tactile feel, viscosity, and stability can be obtained.

Such an emulsion structure can be verified by observation under a polarization microscope. The size of the discontinuous phase refers to the average major axis length of particles of the discontinuous phase that can be verified by observation under a polarization microscope. The number of particles of the discontinuous phase to be verified may be 10 or more, 50 or more, or 100 or more.

Alternatively, the size of the discontinuous phase of the above-described emulsion composition, i.e., the particle size distribution, the median diameter, and the average particle size of the oil phase in the O/W composition, can be measured using a laser diffraction/scattering-type particle size distribution analyzer or a measuring device based on dynamic light scattering method. In the case of performing the measurement using a laser diffraction/scattering-type particle size distribution analyzer, although the analysis conditions are not limited, it is preferred to perform the analysis on a volume basis.

The content of the oil-and-fat in the emulsion composition is usually 0.1% by mass or more, preferably 0.5% by mass or more, more preferably 1% by mass or more, still more preferably 1.5% by mass or more, especially preferably 2% by mass or more, particularly preferably 2.5% by mass or more, specially preferably 3% by mass or more, most preferably 3.5% by mass or more, particularly most preferably 4% by mass or more, and exceptionally preferably 5% by mass or more, with respect to a total amount of the emulsion composition.

Meanwhile, the content of the oil-and-fat is usually less than 80% by mass, preferably 75% by mass or less, more preferably 70% by mass or less, still more preferably 65% by mass or less, particularly preferably 60% by mass or less, specially preferably 55% by mass or less, most preferably 50% by mass or less, particularly most preferably 45% by mass or less, and exceptionally preferably 40% by mass or less.

The ratio of water and the oil-and-fat is usually 950:1 to 1:4, preferably 95:1 to 1:4, more preferably 95:1 to 1:3, still more preferably 95:1 to 1:1, and especially preferably 95:1 to 4:1, in terms of mass ratio.

In the emulsion composition of the present embodiment, a change in the diameter of the discontinuous phase before and after freezing, or before and after heating of sterilization or the like, is preferably small.

As for the change in the diameter before and after freezing or heating, when the percentage difference of the median diameter (D50) of the emulsion composition after freezing or heating is calculated taking the median diameter (D50) of the emulsion composition before freezing or heating as 100%, the median diameter (D50) after heating is preferably ±80% or less, more preferably ±75% or less, still more preferably ±70% or less, especially preferably ±65% or less, particularly preferably ±60% or less, specially preferably ±55% or less, most preferably ±50% or less, particularly most preferably ±49% or less, and exceptionally preferably ±48% or less.

When the percentage difference of the average diameter of the emulsion composition after freezing or heating is calculated taking the average diameter of the emulsion composition before freezing or heating as 100%, the average diameter of the emulsion composition after freezing or heating is preferably ±80% or less, more preferably ±75% or less, still more preferably ±70% or less, especially preferably ±65% or less, particularly preferably ±60% or less, specially preferably ±58% or less, most preferably ±55% or less, particularly most preferably ±53% or less, and exceptionally preferably ±52% or less.

### (Protein Particles or Protein)

The content of the protein particles or the protein in the emulsion composition is usually 0.01% by mass or more, preferably 0.05% by mass or more, more preferably 0.1% by mass or more, still more preferably 0.5% by mass or more, especially preferably 1% by mass or more, particularly preferably 2% by mass or more, and exceptionally preferably 4% by mass or more, with respect to a total amount of the emulsion composition.

Meanwhile, the content of the protein particles or the protein is usually 50% by mass or less, preferably 40% by mass or less, more preferably 30% by mass or less, still more preferably 20% by mass or less, especially preferably 10% by mass or less, and particularly preferably 8% by mass or less.

In the emulsion composition according to the present embodiment, a mass ratio of the content of the protein particles or the protein with respect to the content of the oil-and-fat is not limited; however, it is usually 10 or lower, preferably 8 or lower, more preferably 7 or lower, still more preferably 6 or lower, especially preferably 5 or lower, particularly preferably 4 or lower, specially preferably 3 or lower, most preferably 2 or lower, particularly most preferably 1.5 or lower, and exceptionally preferably 1 or lower.

In the emulsion composition according to the present embodiment, the size of the protein particles or protein existing in the interface between water and the oil-and-fat is usually 0.005 µm or more, preferably 0.01 µm or more, more preferably 0.05 µm or more, still more preferably 0.1 µm or more, and especially preferably 0.2 µm or more.

Meanwhile, the size of the protein particles or protein is usually 500 µm or less, preferably 250 µm or less, more preferably 100 µm or less, still more preferably 50 µm or less, especially preferably 20 µm or less, particularly preferably 5 µm or less, specially preferably 3 µm or less, most preferably 1 µm or less, particularly most preferably 0.9 µm or less, and exceptionally preferably 0.5 µm or less.

The protein particles or protein existing in the interface between water and the oil-and-fat may be in a state of maintaining a size and a shape in the dispersion medium, or may be in a state of being layered or aggregated in the interface. The protein particles or protein may also be in a state of being densely adsorbed to the interface between water and the oil-and-fat or being layered by intermolecular interaction, i.e., in the form of a coating film.

In the state of a coating film, the thickness thereof is defined as the above-described size.

In the above-described range, the protein particles or protein cause no discomfort in the mouth and do not deteriorate the texture.

The size of the protein particles existing in the interface between water and the oil-and-fat is preferably observed in an image obtained by, for example, magnifying an image of the particles that is taken under an optical microscope or a scanning electron microscope (SEM). The number of the particles to be observed may be 5 or more, 20 or more, 50 or more, or 100 or more.

### (Other Components)

The emulsion composition according to the present embodiment may further contain, for example, a surfactant, a milk-derived component, a starch, a sweetener, a stabilizer, a flavoring agent, a colorant, a salt, and an organic acid, within a range that does not impair the effects of the present invention. The emulsion composition according to the present embodiment may also contain an active component that can be expected to exert a desired physiological effect in a living body.

The emulsion composition according to the present embodiment exhibits excellent emulsion stability and/or excellent dispersibility without adding thereto a conventional surfactant. Accordingly, the content of the surfactant may be 0% by mass with respect to a total amount of the composition; however, the composition may contain a surfactant if necessary.

When a surfactant is incorporated, it is preferably used as a substance for surface modification of the protein particles (surface wettability controlling substance). The type of the surfactant is not particularly limited, and examples of the surfactant include an amphiphilic substance having a hydrophobic segment and a hydrophilic segment. The examples of the surfactant also include an ionic surfactant, and a nonionic surfactant.

Thereamong, a nonionic surfactant is preferred since it is hardly affected by pH and salts, and its use is thus not limited.

The content of the surfactant is usually 5% by mass or less, preferably 1% by mass or less, more preferably 0.5% by mass or less, still more preferably 0.2% by mass or less, especially preferably 0.1% by mass or less, particularly preferably 0.05% by mass or less, specially preferably 0.01% by mass or less, most preferably 0.005% by mass or less, particularly most preferably 0.001% by mass or less, and exceptionally preferably 0.0005% by mass or less, with respect to a total amount of the emulsion composition.

By controlling the content of the surfactant to be in this range, the bitterness/taste derived from the surfactant can be avoided. Further, by controlling the content of the surfactant to be in this range, the thermodynamic instability observed in emulsification with the surfactant can be reduced, so that the long-term emulsion stability, heat resistance, and freezing resistance can be improved.

Examples of the milk-derived component include: liquid materials, such as cow milk, processed milk, skimmed milk, fresh cream, whey, buttermilk, sweetened condensed milk, and sugar-free condensed milk; and powdered milk products, such as whole milk powder, skimmed milk powder, modified milk powder, powdered cream, powdered whey, and buttermilk powder. The milk-derived component is particularly preferably buttermilk or buttermilk powder.

From the standpoint of not allowing an allergenic substance to be incorporated into the emulsion composition, the milk-derived component preferably contains neither casein nor *β*-lactoglobulin that is highly allergenic, and more preferably contains no milk-derived protein.

The content of the milk-derived component is usually 0.5% by mass or less, preferably 0.2% by mass or less, more preferably 0.1% by mass or less, still more preferably 0.05% by mass or less, especially preferably 0.01% by mass or less, and particularly preferably 0.005% by mass or less, with respect to a total amount of the emulsion composition, and it is especially preferred that the emulsion composition contain substantially no milk-derived component.

The phrase "contain substantially no milk-derived component" means that the emulsion composition of the present embodiment does not contain any milk-derived component other than impurities contained in a purified product prepared from an animal, plant, or fungal raw material. From the standpoint of allergies, it preferred to control the content of the milk-derived component to be in the above-described range.

Examples of the starch include starches derived from potato, waxy potato, wheat, corn, waxy corn, high-amylose corn, sweet potato, rice, waxy rice, cassava, kudzu, dogtooth violet, mung bean, sago palm, bracken fern, and *Cardiocrinum cordatum* var. *glehnii.*

Examples of the sweetener include: sugars, such as monosaccharides, disaccharides, and oligosaccharides; sugar alcohols, such as monosaccharide alcohols, disaccharide alcohols, trisaccharide alcohols, tetra- and higher-saccharide alcohols, and powder reduced maltose syrups; and high-intensity sweeteners, such as aspartame and neotame.

Examples of the stabilizer include galactomannan, xanthan gum, carrageenan, gum arabic, tamarind gum, gellan gum, glucomannan, and cellulose.

Examples of the flavoring agent include: vanilla flavoring agents, such as vanilla essence; and milk flavoring agents, such as milk flavor and butter flavor. A milk flavoring agent is particularly preferred.

Examples of the colorant include cacao pigment, *β-*carotene, annatto pigment, red pepper pigment, turmeric pigment, oil red pigment, paprika pigment, naphthol yellow pigment, and riboflavin butyrate (VB2).

Examples of the salt include: chlorides, such as sodium chloride, potassium chloride, and magnesium chloride; carbonates, such as sodium carbonate, potassium carbonate, and calcium carbonate; bicarbonates, such as sodium bicarbonate; phosphates, such as disodium phosphate, trisodium phosphate, and dipotassium phosphate; sodium polyphosphate; citrates, such as sodium citrate; and sodium lactate.

Magnesium-containing salts are particularly preferred, and examples of magnesium-containing salts that can be used in food applications include whey minerals, magnesium chloride, magnesium oxide, magnesium carbonate, magnesium sulfate, bittern (crude seawater magnesium chloride), dolomite, unrefined salt, magnesium stearate, magnesium hydrogen phosphate, trimagnesium phosphate, magnesium silicate, magnesium hydroxide, magnesium acetate, magnesium citrate, magnesium malate, magnesium benzoate, magnesium gluconate, magnesium L-glutamate, sepiolite, talc, and phytin.

Examples of the organic acid include fumaric acid, succinic acid, citric acid, tartaric acid, diacetyl tartaric acid, malic acid, adipic acid, glutaric acid, and maleic acid.

Examples of the active component that can be expected to exert a desired physiological effect in a living body include fats, trace elements, vitamins, amino acids, minerals, and medicinal ingredients derived from natural or synthetic compounds.

### (Method of Producing Emulsion Composition)

The emulsion composition according to the present embodiment can be produced by a known method. Examples of the known method include a method of mixing water, an oil-and-fat, and protein particles or a protein, along with other components if necessary, and subsequently stirring the resulting mixture using an arbitrary stirring device.

Specifically, the emulsion composition according to the present embodiment can be obtained by, but not limited to, the following method.
In the case of using protein particles:
a production method that includes the step A1 of dispersing the protein particles in water, and the step A2 of mixing the thus obtained aqueous dispersion of the protein particles with an oil-and-fat and stirring the resulting mixture; or
a production method that includes the step A1' of dispersing the protein particles in an oil-and-fat, and the step A2' of mixing the thus obtained oil-and-fat dispersion of the protein particles with water and stirring the resulting mixture.

In the case of using a protein:
a production method that includes the step B1 of dispersing the protein in water to obtain an aqueous dispersion of protein particles, and the step B2 of mixing the thus obtained aqueous dispersion of protein particles with an oil-and-fat and stirring the resulting mixture; or
a production method that includes the step B1' of dispersing the protein in an oil-and-fat to obtain an oil-and-fat dispersion of protein particles, and the step B2' of mixing the thus obtained oil-and-fat dispersion of protein particles with water and stirring the resulting mixture.

The steps A1 and A1' as well as the steps B1 and B1' may be performed at normal temperature and normal pressure, or may be performed in a heated and/or high-pressure condition. Stirring may also be performed to prepare a dispersion. The stirring speed and the stirring time are not limited; however, the stirring speed may be usually 10 rpm to 20,000 rpm, and the stirring time is usually 10 seconds to 5 hours. The stirring speed and the stirring time may be changed in a stepwise manner.

Examples of the stirring device include high-pressure homogenizers, paddle mixers, homogenizers, ultrasonic homogenizers, colloid mills, kneaders, in-line mixers, static mixers, ONLATOR, and homomixers.

From the standpoint of performing sufficient stirring with a low energy and at a low cost, it is preferred to use a homomixer, a paddle mixer, or a homogenizer. It is more preferred to use a homomixer since it has a large convection area and can uniformly stir the whole mixture. A combination of different stirring devices may be used as well.

The steps A2 and A2' as well as the steps B2 and B2' are the steps of preparing an emulsion composition. In these steps, the stirring of the mixture is typically performed in a heated condition so as to sufficiently melt the oil-and-fat, usually at 10°C to 100°C, preferably at 20°C to 90°C, more preferably at 30°C to 90°C, still more preferably at 40°C to 90°C, particularly preferably at 50°C to 90°C, and exceptionally preferably at 60°C to 90°C.

The stirring speed may be usually 10 rpm to 20,000 rpm, and the stirring time is usually 10 seconds to 60 minutes.

The stirring conditions are not limited, and a more stable emulsion composition can be formed by changing the stirring speed and the stirring time in a stepwise manner.

Specifically, by finely dispersing oil droplets by high-speed stirring in a first step, and performing stirring of the subsequent second step at a lower speed than the stirring of the first step, adsorption of the protein particles or the protein to the interface between water and the oil-and-fat is facilitated, as a result of which emulsification can be stabilized.

Further, during the stirring of the second step, not only defective adsorption of the protein particles or the protein to the interface between water and the oil-and-fat, which is caused by a shear force originating from the device that is generated during the high-speed stirring of the first step, can be inhibited, but also desorption of the once adsorbed protein particles or protein from the interface can be inhibited.

In the case of changing the stirring conditions in a stepwise manner, the stirring speed in the first step is usually 3,000 rpm or more, preferably 3,500 rpm or more, more preferably 4,000 rpm or more, still more preferably 4,500 rpm or more, especially preferably 5,000 rpm or more, particularly preferably 5,500 rpm or more, specially preferably 6,000 rpm or more, most preferably 6,500 rpm or more, particularly most preferably 7,000 rpm or more, and exceptionally preferably 8,000 rpm or more.

There is no upper limit on the stirring speed; however, the stirring speed is usually 25,000 rpm or less, preferably 20,000 rpm or less, more preferably 19,000 rpm or less, still more preferably 18,000 rpm or less, especially preferably 17,000 rpm or less, particularly preferably 16,000 rpm or less, specially preferably 15,000 rpm or less, most preferably 14,000 rpm or less, particularly most preferably 12,000 rpm or less, and exceptionally preferably 10,000 rpm or less.

The stirring time in the first step is usually 5 seconds or longer, preferably 10 seconds or longer, more preferably 15 seconds or longer, still more preferably 20 seconds or longer, especially preferably 25 seconds or longer, particularly preferably 30 seconds or longer, specially preferably 40 seconds or longer, most preferably 45 seconds or longer, particularly most preferably 50 seconds or longer, and exceptionally preferably 1 minute or longer.

There is no upper limit on the stirring time; however, the stirring time is usually 1 hour or shorter, preferably 30 minutes or shorter, more preferably 15 minutes or shorter, and still more preferably 5 minutes or shorter.

In the case of changing the stirring conditions in a stepwise manner, the stirring speed in the second step is usually 10 rpm or more, preferably 50 rpm or more, more preferably 100 rpm or more, still more preferably 200 rpm or more, especially preferably 400 rpm or more, particularly preferably 500 rpm or more, specially preferably 1,000 rpm or more, most preferably 1,500 rpm or more, particularly most preferably 2,000 rpm or more, and exceptionally preferably 2,500 rpm or more.

There is no upper limit on this stirring speed; however, the stirring speed is usually 10,000 rpm or less, preferably 9,000 rpm or less, more preferably 8,000 rpm or less, still more preferably 7,000 rpm or less, especially preferably 6,000 rpm or less, particularly preferably 5,500 rpm or less, specially preferably 5,000 rpm or less, most preferably 4,500 rpm or less, particularly most preferably 4,000 rpm or less, and exceptionally preferably 3,000 rpm or less.

The stirring time is not particularly limited; however, from the standpoint of facilitating the adsorption of the protein particles or the protein to the interface between water and the oil-and-fat, the stirring time is usually 5 seconds or longer, preferably 10 seconds or longer, more preferably 15 seconds or longer, still more preferably 30 seconds or longer, especially preferably 45 seconds or longer, particularly preferably 1 minute or longer, specially preferably 3 minutes or longer, most preferably 5 minutes or longer, particularly most preferably 10 minutes or longer, and exceptionally preferably 20 minutes or longer.

After the preparation of the emulsion composition, a sterilization treatment may be performed as well.

The temperature of the sterilization treatment is usually 60°C or higher, preferably 65°C or higher, more preferably 70°C or higher, still more preferably 75°C or higher, especially preferably 80°C or higher, particularly preferably 85°C or higher, specially preferably 90°C or higher, most preferably 95°C or higher, particularly most preferably 100°C or higher, and exceptionally preferably 110°C or higher.

An upper limit of the temperature of the sterilization treatment is usually 160°C or lower, preferably 158°C or lower, more preferably 155°C or lower, still more preferably 153°C or lower, especially preferably 150°C or lower, particularly preferably 148°C or lower, specially preferably 145°C or lower, most preferably 143°C or lower, particularly most preferably 140°C or lower, and exceptionally preferably 135°C or lower.

The duration of the sterilization treatment is usually 0.01 minutes or longer, preferably 0.012 minutes or longer, more preferably 0.015 minutes or longer, still more preferably 0.017 minutes or longer, especially preferably 0.02 minutes or longer, particularly preferably 0.022 minutes or longer, specially preferably 0.025 minutes or longer, most preferably 0.027 minutes or longer, particularly most preferably 0.03 minutes or longer, and exceptionally preferably 0.035 minutes or longer.

An upper limit of the duration of the sterilization treatment is usually 60 minutes or shorter, preferably 58 minutes or shorter, more preferably 55 minutes or shorter, still more preferably 52 minutes or shorter, especially preferably 50 minutes or shorter, particularly preferably 45 minutes or shorter, specially preferably 40 minutes or shorter, most preferably 35 minutes or shorter, and particularly most preferably 30 minutes or shorter.

A sterilization method is not particularly limited, and examples thereof include UHT sterilization, retort sterilization, and Joule sterilization.

The UHT sterilization can be performed by a known method, for example, a direct heating method such as a steam injection method in which steam is directly blown into a composition or a steam infusion method in which a composition is sprayed into steam and thereby heated, or an indirect heating method using a surface heat exchanger such as a plate-type or tube-type heat exchanger. For example, a plate-type sterilization device can be used.

### (pH)

The pH of the emulsion composition is not particularly limited, and a suitable pH may be selected in accordance with the intended use. For example, when the emulsion composition is used in a food application, the emulsion composition may have any pH as long as it is edible and drinkable; however, a lower limit value of the pH is usually higher than 1, preferably 2 or higher, more preferably 3 or higher, still more preferably 4 or higher, especially preferably 5 or higher, particularly preferably 5.5 or higher, specially preferably 6 or higher, most preferably 6.2 or higher, particularly most preferably 6.3 or higher, and exceptionally preferably 6.5 or higher.

An upper limit value of the pH is usually 13 or lower, preferably 12 or lower, more preferably 10 or lower, still more preferably 9.5 or lower, especially preferably 9 or lower, particularly preferably 8.5 or lower, specially preferably 8 or lower, most preferably 7.8 or lower, particularly most preferably 7.5 or lower, and exceptionally preferably 7.4 or lower.

The pH of the emulsion composition may be around the isoelectric point of the protein particles, the protein, or the raw material protein constituting the protein particles or the protein; however, from the standpoint of avoiding the generation of coarse aggregates and precipitates, the pH of the emulsion composition is usually apart from the isoelectric point by at least 0.5, and preferably by at least 1.

By controlling the pH to be in this range, a higher emulsion stability can be maintained. Further, by controlling the pH to be in this range, aggregation of the protein particles and/or the protein can be inhibited and the dispersed phase can be maintained at a preferred size (oil droplet size), so that a good texture in eating can be maintained.

### <Use>

The protein particles, the protein, and the emulsion composition according to embodiments of the present invention may be used in pharmaceutical products, cosmetic products, foods, feeds, diagnostic agents, carriers for drug delivery systems (DDS), cleaners, coating agents, surface treatment agents, toiletry products, personal care products, and the like, and can be used for, for example, oral ingestion or transdermal absorption. The protein particles, the protein, and the emulsion composition can also be used as production intermediates of the above.

In the case of using the protein particles, the protein, and the emulsion composition according to embodiments of the present invention in various products, the protein particles, the protein, and the emulsion composition may be produced in a grade appropriate for the specification of the respective products.

For example, the protein particles, the protein, and the food-grade emulsion composition can be used in various foods (specific use are described below).

The protein particles, the protein, and the cosmetic-grade emulsion composition can be used in cosmetic products.

The protein particles, the protein, and the pharmaceutical-grade emulsion composition can be used in pharmaceutical products.

The protein particles, the protein, and the personal care product-grade emulsion composition can be used in personal care products.

Specific examples of the use for oral ingestion include: foods and drinks, such as beverages, liquid foods, creamy foods, and animal-derived food substitutes; retort processed dietary supplements; functional foods, such as liquid diets; oral vaccines; processed flour products, such as breads and noodles; processed oil-and-fat products, such as fat spreads and flour pastes; various sauces and soups, such as curry, coffee creamer, mayonnaise, dressing, mousse, pasta sauce, stew, demi-glace sauce, white sauce, and tomato sauce; retort processed foods and composite seasonings, such as Chinese flavor seasonings and rice bowl ingredients; confectioneries and desserts, such as yogurts, cheese, ice creams, creams, caramels, candies, chewing gum, chocolates, cookies, biscuits, cakes, pies, snacks, crackers, Japanese sweets, rice confectionery, bean confectionery, jellies, and puddings; processed livestock products, such as hamburg steaks, meatballs, and canned seasoned meat; frozen foods; refrigerated foods; pre-cooked or ready-to-cool meals, such as packed or in-store prepared daily dishes; instant foods, such as instant noodles, cup noodles, and instant soups and stews; fortified foods; foods and drinks, such as liquid foods, high-calorie foods, and nutritional products for infants; and tube feeding preparations.

Particularly, beverages and foods such as liquid foods are preferred, and examples of the beverages include milk beverages, soup beverages, coffee beverages, cocoa beverages, tea beverages (e.g., black tea, green tea, and Chinese tea), bean or cereal beverages, and acidic beverages. Thereamong, milk beverages, coffee beverages, and tea beverages are preferred.

The "animal-derived food substitutes" refer to emulsion compositions that can each be a substitute for animal-derived foods such as cow milk from the standpoint of taste, flavor, and physical properties. Examples of the animal-derived food substitutes include: milk substitutes; and dairy product-like foods, dairy product-like beverages, and concentrated milk that do not contain any animal-derived dairy product. Milk substitutes are also called "plant milk". Examples of plant milk include almond beverages, oat beverages, coconut beverages, rice milk, cashew nut milk, hemp milk, pea milk, walnut beverages, soy milk, pistachio milk, barley milk, macadamia milk, and fruit juice beverages.

The emulsion composition according to one embodiment of the present invention can be used as a production intermediate of a food such as yogurt or ice cream. Examples of the above-mentioned physical properties include the particle size distribution of the oil droplets in the composition, the viscosity, the pH, the stability of the emulsion, and the appearance. Further, the emulsion composition for food can be preferably used for packaged beverages, such as canned beverages, plastic bottled beverages, carton beverages, and glass bottled beverages.

Other examples of the animal-derived food substitutes include meat substitutes. The "meat substitutes" refer to foods that do not contain any animal-derived product or raw material such as a dairy product, and are produced from raw materials other than meat. Meat substitutes are also called "meat alternatives", "meat replacements", "mock meat", "artificial meat", and "imitation meat".

### EXAMPLES

The present invention will now be described in more detail by way of Examples; however, it is needless to say that the scope of the present invention is not limited to the modes shown in the below-described Examples. In the below-described Examples, unless otherwise specified, "part(s)" and "%" mean "part(s) by mass" and "% by mass", respectively.

The methods used for measuring the physical properties in Examples are as follows.

### <1. Measurement of Particle Size Distribution of Protein Particles or Emulsions>

The size of oil droplets constituting the inner phase of an emulsion composition or protein particles was measured by a batchwise method using a laser diffraction/scattering-type particle size distribution analyzer LA-950 (manufactured by HORIBA, Ltd.). The substance to be measured was analyzed, assuming the refractive index thereof as 1.60.

The average particle size and the median diameter of a protein or protein particles were calculated by analyzing based on number. The average particle size and the median diameter of oil droplets constituting the inner phase of an emulsion composition were calculated by analyzing based on number and based on volume.

### <2. Measurement of pH>

The pH was measured using LAQUA PH/ION METER F-72 manufactured by Horiba, Ltd.

### <3. Measurement by Gel Filtration Chromatography (GPC)>

The molecular weight of protein particles was analyzed by the following method.

First, 1 mL of the below-described 5% protein particle aqueous dispersion was dispensed into an Eppendorf tube and centrifuged at 10,000 rpm for 10 minutes, and the resulting precipitate was separated. The centrifugal supernatant was recovered and filtered through a 0.45-µm cartridge filter, and 0.05 mL of the thus obtained filtrate was evaporated to dryness using a centrifugal evaporator.

This dried product was re-dissolved or re-dispersed in 0.25 mL of a GPC eluent [Dulbecco's PBS(-) aqueous solution], and the resulting supernatant was promptly (within 3 hours) subjected to gel filtration chromatography measurement. The equipment conditions are as follows.
Equipment: Shimadzu LC10A, Shimadzu GPC analysis software (LC solution GPC)
Column: TSKgel G5000PW × 1, G3000PW × 1, G2500PW × 1 that were sequentially connected (manufactured by Tosoh Corporation, 7.8 × 300 mm), 40°C
Mobile phase: Dulbecco's PBS(-) aqueous solution, 1 mL/min
Detection: differential refractive index (RI) detector
Sample: 50-µL injection

### <4. Analysis of Oil-and-Fat>

Tests for analyzing the acid value, the saponification value, and the slip melting point of an oil-and-fat were conducted in accordance with the methods described in the Standard Methods for the Analysis of Fats, Oils, and Related Materials (Japan Oil Chemists' Society).

### <Raw Materials>

The physical properties of each raw material were as follows.
- hydrogenated coconut oil: solid at 25°C, acid value = 0.47, saponification value = 255, slip melting point = 28.8°C

### <Preparation of Protein Particles>

Protein particles were prepared by the below-described steps using a pea protein (NUTRALYS (registered trademark) S85F, a plant protein extracted from yellow pea *(Pisum sativum))* as a protein and demineralized water as water in a total amount of 100 parts. Table 1 shows the added amount (parts) of each component used in Examples and Comparative Examples.

Each protein particle aqueous dispersion was stored in a refrigerator at 4°C. Further, each protein particle powder was stored at room temperature.

As a stirrer, T.K. ROBOMIX manufactured by PRIMIX Corporation (equipped with T.K. HOMOGENIZING MIXER MARK II Model 2.5 as a stirring unit), or HOMOGENIZING MIXER MARK II (Model 2.5) was used. These stirrers are hereinafter each referred to as "homomixer".

### (Example 1)

In a container, 95 parts of demineralized water and 5 parts of the pea protein were fractionated and mixed. The resulting mixture was heat-treated at 121°C for about 30 minutes using an autoclave or a retort sterilizer, and subsequently allowed to cool by immersing the container in a 20°C water bath, whereby a protein particle aqueous dispersion A1 was obtained.

Next, the thus obtained protein particle aqueous dispersion A1 was fractionated into another container, and then stirred at room temperature and 8,000 rpm for 5 minutes using a homomixer, whereby a protein particle aqueous dispersion A1-1 was obtained.

Further, 20 g of the protein particle aqueous dispersion A1 was fractionated into a container, and then rapidly cooled and frozen by immersing the bottom of the container in liquid nitrogen for at least 5 minutes. Thereafter, the resultant was freeze-dried under reduced pressure for 2 days using a square dry chamber DRC-1100 (EYELA, manufactured by Tokyo Rikakikai Co., Ltd.) and a freeze dryer FDU-2100 (EYELA, manufactured by Tokyo Rikakikai Co., Ltd.), whereby a protein particle powder A2 was obtained. The shelf temperature of the square dry chamber was set to -5°C in advance.

As the treatment using an autoclave, a high-temperature high-pressure treatment was performed at 121°C and 0.12 MPa for 30 minutes using HICLAVE HG-80 manufactured by Hirayama Manufacturing Corp.

The treatment using a retort sterilizer was performed using RCS-40RTGN-FAM manufactured by Hisaka Works, Ltd., with the device conditions being set at 121.1°C and 29 minutes (The chamber internal pressure was indicated to be 0.197 MPa when the temperature reached 119.5°C. Since water was introduced to the chamber to operate this sterilizer, the indicated pressure corresponded to 0.204 MPa at a temperature of 121°C, taking into account the saturated vapor pressure curve of water). After the completion of the 29-minute treatment at 121°C using the retort sterilizer, the retort sterilizer was operated at 0.180 MPa during the subsequent cooling. The above-described mixture was filled into a can container, which was subsequently hermetically sealed and subjected to the treatment using the retort sterilizer.

### (Example 2)

In a container, 95 parts of demineralized water and 5 parts of the pea protein were fractionated and mixed. The resulting mixture was heat-treated at 121°C and 0.12 MPa for 60 minutes using an autoclave (HICLAVE HG-80, manufactured by Hirayama Manufacturing Corp.), and subsequently allowed to cool by immersing the container in a 20°C water bath, whereby a protein particle aqueous dispersion B1 was obtained.

Next, the thus obtained protein particle aqueous dispersion B1 was fractionated into another container, and then stirred at room temperature and 8,000 rpm for 5 minutes using a homomixer, whereby a protein particle aqueous dispersion B1-1 was obtained.

Further, the protein particle aqueous dispersion B1 was freeze-dried to obtain a protein particle powder B2.

### (Comparative Example 1)

A protein particle aqueous dispersion C1, a protein particle aqueous dispersion C1-1, and a protein particle powder C2 were obtained in the same manner as in Example 1, except that the prepared mixture was not heat-treated.

### (Comparative Example 2)

A protein particle aqueous dispersion D1, a protein particle aqueous dispersion D1-1, and a protein particle powder D2 were obtained in the same manner as in Example 1, except that the heat treatment of the prepared mixture was changed to a 30-minute heat treatment at 90°C using a water bath.

### (Comparative Example 3)

A protein particle aqueous dispersion E1, a protein particle aqueous dispersion E1-1, and a protein particle powder E2 were obtained in the same manner as in Example 1, except that the heat treatment of the prepared mixture was changed to a 30-minute heat treatment at 95°C using a water bath.

### (Comparative Example 4)

In a container, 85 parts of demineralized water and 15 parts of the pea protein were fractionated and mixed. The resulting mixture was heat-treated at 90°C for 60 minutes using a water bath, and subsequently allowed to cool by immersing the container in a 20°C water bath.

Demineralized water was added to this mixture such that the pea protein concentration was adjusted to be 5 parts, after which the mixture was then stirred at 8,000 rpm for 5 minutes using a homomixer, whereby a protein particle aqueous dispersion F1 was obtained.

Further, the thus obtained protein particle aqueous dispersion F1 was freeze-dried to obtain a protein particle powder F2.

Table 1 shows the particle size distribution measured for each aqueous dispersion. Further, Table 2 shows the measurement results obtained by gel filtration chromatography.

**Table 1**

| | Exampl e 1 | Exampl e 1 | Exampl e 2 | Exampl e 2 | Compa rative Exampl e 1 | Compa rative Exampl e 1 | Compa rative Exampl e 2 | Compa rative Exampl e 2 | Compa rative Exampl e 3 | Compa rative Exampl e 3 | Compa rative Exampl e 4 |
|---|---|---|---|---|---|---|---|---|---|---|---|
| Protein particle aqueous dispersion | A1 | A1-1 | B1 | B1-1 | C1 | C1-1 | D1 | D1-1 | E1 | E1-1 | F1 |
| Heat treatment conditions Temperature/Time | 121°C /30min | 121°C /30min | 121°C /60min | 121°C /60min | Not heated | Not heated | 90°C /30min | 90°C /30min | 95°C /30min | 95°C /30min | 90°C /60min |
| Pea protein concentration at the time of heat treatment [parts] | 5 | 5 | 5 | 5 | 5 | 5 | 5 | 5 | 5 | 5 | 15 |
| Average particle size of protein particles in water [µm] | 0.0904 | 0.111 | 0.0903 | 0.0952 | 14.7 | 10.7 | 8.91 | 0.104 | 0.164 | 0.109 | 0.0942 |
| Median diameter of protein particles in water [µm] | 0.0840 | 0.106 | 0.0837 | 0.0917 | 11.6 | 9.55 | 7.95 | 0.0978 | 0.158 | 0.103 | 0.0879 |

**Table 2**

| | Example 1 | Example 2 | Comparati ve Example 1 | Comparati ve Example 2 | Comparati ve Example 3 | Comparati ve Example 4 |
|---|---|---|---|---|---|---|
| Protein particle aqueous dispersion | A1-1 | B1-1 | C1-1 | D1-1 | E1-1 | F1 |
| Heat treatment conditions Temperature/Time | 121°C /30min | 121°C /60min | Not heated | 90°C /30min | 95°C /30min | 90°C /60min |
| Pea protein concentration at the time of heat treatment [parts] | 5 | 5 | 5 | 5 | 5 | 15 |
| (B):weight-average molecular weight Mw | 79,000 | 110,000 | 11,000 | 17,000 | 27,000 | 20,000 |
| (C):number-average molecular weight Mn | 5,400 | 5,700 | 2,600 | 2,900 | 3,500 | 2,600 |
| Mw/Mn | 14.73 | 19.14 | 4.30 | 6.01 | 7.71 | 7.50 |

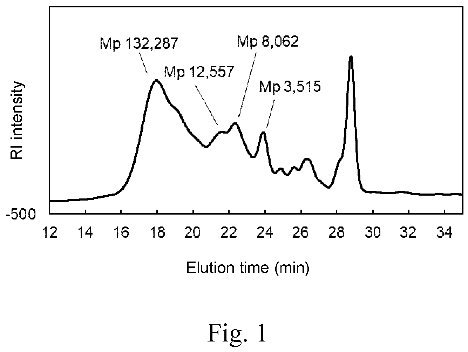

For the protein particle aqueous dispersions A1-1, B1-1, C1-1, D1-1, E1-1, and F1 that were obtained in Examples 1 and 2 and Comparative Examples 1 to 4, the elution curves of gel filtration chromatography are shown in FIGs. 1 to 6.

In the chart of FIG. 1, a peak (Mp 132,287) of a molecular weight of 132,287, a peak (Mp 12,557) of a molecular weight of 12,557, a peak (Mp 8,062) of a molecular weight of 8,062, and a peak (Mp 3,515) of a molecular weight of 3,515, which molecular weights are in terms of polyethylene glycol, were confirmed.

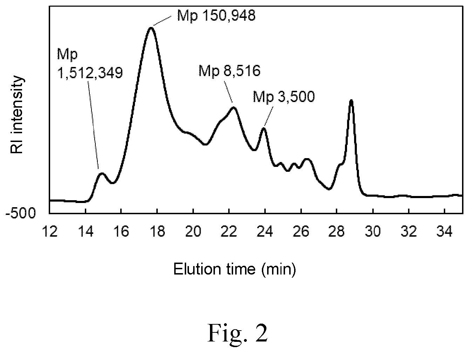

In the chart of FIG. 2, a peak (Mp 1,512,349) of a molecular weight of 1,512,349, a peak (Mp 150,948) of a molecular weight of 150,948, a peak (Mp 8,516) of a molecular weight of 8,516, and a peak (Mp 3,500) of a molecular weight of 3,500, which molecular weights are in terms of polyethylene glycol, were confirmed.

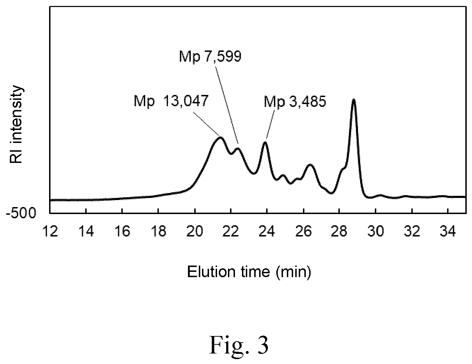

In the chart of FIG. 3, a peak (Mp 13,047) of a molecular weight of 13,047, a peak (Mp 7,599) of a molecular weight of 7,599, and a peak (Mp 3,485) of a molecular weight of 3,485, which molecular weights are in terms of polyethylene glycol, were confirmed.

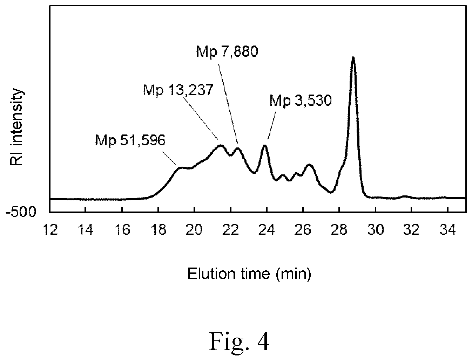

In the chart of FIG. 4, a peak (Mp 51,596) of a molecular weight of 51,596, a peak (Mp 13,237) of a molecular weight 13,237, a peak (Mp 7,880) of a molecular weight of 7,880, and a peak (Mp 3,530) of a molecular weight of 3,530, which molecular weights are in terms of polyethylene glycol, were confirmed.

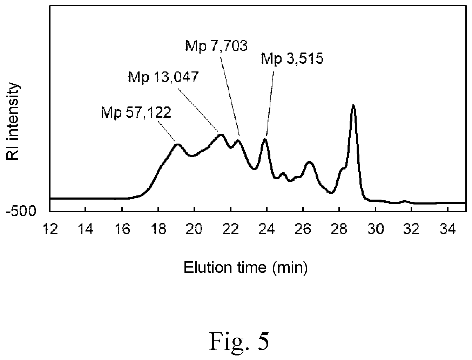

In the chart of FIG. 5, a peak (Mp 57,122) of a molecular weight of 57,122, a peak (Mp 13,047) of a molecular weight of 13,047, a peak (Mp 7,703) of a molecular weight of 7,703, and a peak (Mp 3,515) of a molecular weight of 3,515, which molecular weights are in terms of polyethylene glycol, were confirmed.

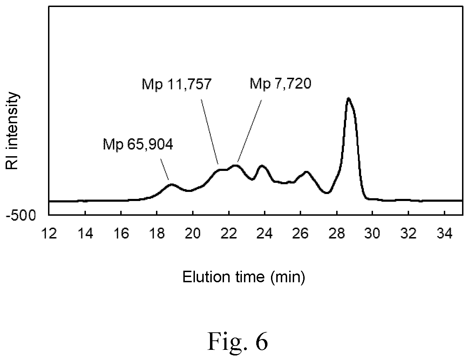

In the chart of FIG. 6, a peak (Mp 65,904) of a molecular weight of 65,904, a peak (Mp 11,757) of a molecular weight of 11,757, and a peak (Mp 7,720) of a molecular weight of 7,720, which molecular weights are in terms of polyethylene glycol, were confirmed.

As compared to the protein particle aqueous dispersions C1-1 (Comparative Example 1), D1-1 (Comparative Example 2), E1-1 (Comparative Example 3), and F1 (Comparative Example 4), the protein particle aqueous dispersions A1-1 (Example 1) and B1-1 (Example 2) were eluted in a shorter time, and had a structure with a higher molecular weight. Therefore, it is seen that a high-molecular-weight material is efficiently formed by the above-described heat treatment step.

### <Preparation of Oil-in-Water Emulsion Compositions>

### (Example 3)

The protein particle aqueous dispersion A1 and hydrogenated coconut oil heated to 60°C were fractionated into a container at the mass ratio shown in Table 3. These materials were mixed at 60°C, and subsequently stirred at 60°C and 10,000 rpm for 1 minute and then at 3,000 rpm for 20 minutes using a homomixer, whereby an oil-in-water emulsion composition A1 was obtained.

Next, the protein particle aqueous dispersion A1-1 and hydrogenated coconut oil heated to 60°C were fractionated into a container at the mass ratio shown in Table 3. These materials were mixed at 60°C, and subsequently stirred at 60°C and 10,000 rpm for 1 minute and then at 3,000 rpm for 20 minutes using a homomixer, whereby an oil-in-water emulsion composition A1-1 was obtained.

### (Example 4)

An oil-in-water emulsion composition B1 and an oil-in-water emulsion composition B1-1 were obtained in the same manner as in Example 3, except that the protein particle aqueous dispersions B1 and B1-1 were used, respectively.

### (Comparative Example 5)

An oil-in-water emulsion composition C1 and an oil-in-water emulsion composition C1-1 were obtained in the same manner as in Example 3, except that the protein particle aqueous dispersions C1 and C1-1 were used, respectively.

### (Comparative Example 6)

An oil-in-water emulsion composition D1-1 was obtained in the same manner as in Example 3, except that the protein particle aqueous dispersion D1-1 was used.

### (Comparative Example 7)

An oil-in-water emulsion composition E1-1 was obtained in the same manner as in Example 3, except that the protein particle aqueous dispersion E1-1 was used.

### (Comparative Example 8)

An oil-in-water emulsion composition F1 was obtained in the same manner as in Example 3, except that the protein particle aqueous dispersion F1 was used.

**Table 3**

| | | Exampl e 3 | Exampl e 3 | Exampl e 4 | Exampl e 4 | Compa rative Exampl e 5 | Compa rative Exampl e 5 | Compa rative Exampl e 6 | Compa rative Exampl e 7 | Compa rative Exampl e 8 |
|---|---|---|---|---|---|---|---|---|---|---|
| Emulsion composition | | A1 | A1-1 | B1 | B1-1 | C1 | C1-1 | D1-1 | E1-1 | F1 |
| | A1 | 95 | 0 | 0 | 0 | 0 | 0 | 0 | 0 | 0 |
| | A1-1 | 0 | 95 | 0 | 0 | 0 | 0 | 0 | 0 | 0 |
| | B1 | 0 | 0 | 95 | 0 | 0 | 0 | 0 | 0 | 0 |
| Aqueous phase Protein particle aqueous dispersion [parts] | B1-1 | 0 | 0 | 0 | 95 | 0 | 0 | 0 | 0 | 0 |
| | C1 | 0 | 0 | 0 | 0 | 95 | 0 | 0 | 0 | 0 |
| | C1-1 | 0 | 0 | 0 | 0 | 0 | 95 | 0 | 0 | 0 |
| | D1-1 | 0 | 0 | 0 | 0 | 0 | 0 | 95 | 0 | 0 |
| | E1-1 | 0 | 0 | 0 | 0 | 0 | 0 | 0 | 95 | 0 |
| | F1 | 0 | 0 | 0 | 0 | 0 | 0 | 0 | 0 | 95 |
| Oil phase [parts] | Hydrogenated coconut oil | 5 | 5 | 5 | 5 | 5 | 5 | 5 | 5 | 5 |
| Total amount [parts] | | 100 | 100 | 100 | 100 | 100 | 100 | 100 | 100 | 100 |
| Protein particle concentration in emulsion composition [%] | | 4.75 | 4.75 | 4.75 | 4.75 | 4.75 | 4.75 | 4.75 | 4.75 | 4.75 |
| Protein/oil-and-fat [mass ratio] | | 0.95 | 0.95 | 0.95 | 0.95 | 0.95 | 0.95 | 0.95 | 0.95 | 0.95 |

### <Evaluation of Heat Resistance of Emulsion compositions>

Each of the above-obtained oil-in-water emulsion compositions A1, A1-1, B1, B1-1, C1, and C1-1 in an amount of 20 g was fractionated into a transparent container, and heat-treated in an autoclave at 121°C for 30 minutes with the opening of the container being covered with an aluminum foil.

After heating the autoclave and confirming that the internal temperature of the autoclave was 80°C or lower, the container was taken out and, before the container was cooled to room temperature, the presence or absence of oil phase separation was visually checked from a side of the container and evaluated based on the following criteria.
o: Absence of oil phase separation
×: Presence of oil phase separation
The results are shown in Table 4.

**Table 4**

| | | Example 3 | Example 3 | Example 4 | Example 4 | Comparat ive Example 5 | Comparat ive Example 5 |
|---|---|---|---|---|---|---|---|
| Emulsion composition | | A1 | A1-1 | B1 | B1-1 | C1 | C1-1 |
| Heating at 121°C | Before heating | ○ | ○ | ○ | ○ | ○ | ○ |
| | After heating | ○ | ○ | ○ | ○ | × | ○ |

Focusing on the emulsion compositions C1 and C1-1 according to Comparative Example 5, the emulsion composition C1-1, in which the protein particle aqueous dispersion was preliminarily stirred, was not observed with oil phase separation after being heated at 121°C, and exhibited a good heat resistance; however, the emulsion composition C1, in which the preliminary stirring process was not performed, exhibited poor stability when heated at 121°C. On the other hand, the emulsion compositions A1 and B1 according to Examples 3 and 4 were not observed with oil phase separation after being heated at 121°C, and exhibited a good post-heating emulsion stability, even without the preliminary stirring process.

### <Cooling Resistance of Emulsion Compositions>

The oil-in-water emulsion compositions A1, A1-1, B1, and B1-1 according to Examples 3 and 4 which were emulsified at 60°C were each put into a container, and the container was immersed in a water bath for at least 30 minutes and thereby cooled to a temperature of 25°C. Subsequently, the container was inverted, and the fluidity and the presence or absence of aggregation of each oil-in-water emulsion composition before and after the cooling were visually checked and evaluated based on the following criteria.
o: The emulsion exhibited a good fluidity with no aggregates.
×: The emulsion exhibited a poor fluidity with generation of aggregates.

The results are shown in Table 5.

**Table 5**

| | | Example 3 | Example 3 | Example 4 | Example 4 |
|---|---|---|---|---|---|
| Emulsion composition | | A1 | A1-1 | B1 | B1-1 |
| Cooling to room temperature | Before cooling | ○ | ○ | ○ | ○ |
| | After cooling | ○ | ○ | ○ | ○ |

As apparent from the results shown in Table 5, the emulsion compositions according to one embodiment of the present invention were flowable with no aggregates even after being cooled, and maintained a stable emulsified state even in such a temperature range where the oil-and-fat constituting the discontinuous phase of each emulsion changes from a liquid state to a solid state.

### <Freezing Resistance of Emulsion Compositions>

### (Evaluation 1)

The oil-in-water emulsion compositions A1, A1-1, B1, B1-1, C1, C1-1, D1-1, E1-1, and F1 according to Examples 3 and 4 and Comparative Examples 5 to 8 were each fractionated into a container and frozen in a freezer.

Then, after thawing at room temperature, the container was inverted, and the appearance was visually checked from a side of the container and evaluated based on the following criteria.
∘: No aggregate was visually observed in the emulsion composition.
×: Aggregates were visually observable in the emulsion composition.

The results are shown in Table 6.

**Table 6**

| | | Exampl e 3 | Exampl e 3 | Exampl e 4 | Exampl e 4 | Compa rative Exampl e 5 | Compa rative Exampl e 5 | Compa rative Exampl e 6 | Compa rative Exampl e 7 | Compa rative Exampl e 8 |
|---|---|---|---|---|---|---|---|---|---|---|
| Emulsion composition | | A1 | A1-1 | B1 | B1-1 | C1 | C1-1 | D1-1 | E1-1 | F1 |
| Freezing | Before freezing | ○ | ○ | ○ | ○ | ○ | ○ | ○ | ○ | ○ |
| | After freezing | ○ | ○ | ○ | ○ | × | × | × | × | × |

As apparent from the results shown in Table 6, the emulsion compositions according to one embodiment of the present invention had no change in appearance and maintained a stable emulsified state even in a temperature process where not only the discontinuous phase of each emulsion but also water constituting the continuous phase were frozen and melted (changed in state).

### (Evaluation 2)

The results of measuring the particle size distribution of each emulsion composition before and after freezing are shown in Tables 7 and 8. Those samples that were visually observed with generation of aggregates were diluted with demineralized water, subsequently pipetted vigorously at least 10 times to break loose aggregates, and then measured.

**Table 7**

| | | Example 3 | Example 4 | Comparati ve Example 5 | Comparati ve Example 6 | Comparati ve Example 7 | Comparati ve Example 8 |
|---|---|---|---|---|---|---|---|
| Emulsion composition | | A1-1 | B1-1 | C1-1 | D1-1 | E1-1 | F1 |
| Based on number | Average particle size of oil droplets before freezing [µm] | 1.99 | 1.97 | 2.15 | 2.08 | 2.01 | 1.95 |
| | Average particle size of oil droplets after thawing [µm] | 2.62 | 2.42 | 4.86 | 3.50 | 3.31 | 7.35 |
| | Rate of change in average particle size before and after freezing [%] | +31.8 | +22.9 | +126 | +68.5 | +64.7 | +277 |
| | Median diameter of oil droplets before freezing [µm] | 1.75 | 1.74 | 1.86 | 1.83 | 1.76 | 1.68 |
| | Median diameter of oil droplets after thawing [µm] | 2.27 | 2.05 | 4.19 | 2.70 | 2.81 | 6.60 |
| | Rate of change in median diameter before and after freezing [%] | +30.0 | +17.9 | +125 | +47.5 | +59.6 | +294 |
| Based on volume | Average particle size of oil droplets before freezing [µm] | 4.91 | 4.33 | 4.84 | 4.15 | 4.69 | 7.63 |
| | Average particle size of oil droplets after thawing [µm] | 5.26 | 6.57 | 15.1 | 13.1 | 7.16 | 29.6 |
| | Rate of change in average particle size before and after freezing [%] | +7.12 | +51.9 | +212 | +215 | +52.7 | +288 |
| | Median diameter of oil droplets before freezing [µm] | 3.82 | 3.62 | 4.27 | 3.69 | 3.89 | 4.47 |
| | Median diameter of oil droplets after thawing [µm] | 4.87 | 5.30 | 9.65 | 9.46 | 6.44 | 14.7 |
| | Rate of change in median diameter before and after freezing [%] | +27.4 | +46.5 | +126 | +156 | +65.5 | +229 |

**Table 8**

| | | Example 3 | Example 4 | Comparative Example 5 |
|---|---|---|---|---|
| Emulsion composition | | A1 | B1 | C1 |
| Based on number | Average particle size of oil droplets before freezing [µm] | 2.29 | 2.29 | 3.30 |
| | Average particle size of oil droplets after thawing [µm] | 2.52 | 2.38 | 5.97 |
| | Rate of change in average particle size before and after freezing [%] | +10.0 | +3.93 | +80.9 |
| | Median diameter of oil droplets before freezing [µm] | 1.99 | 2.00 | 2.81 |
| | Median diameter of oil droplets after thawing [µm] | 2.15 | 2.04 | 5.19 |
| | Rate of change in median diameter before and after freezing [%] | +8.04 | +2.00 | +84.7 |
| Based on volume | Average particle size of oil droplets before freezing [µm] | 4.90 | 4.89 | 7.20 |
| | Average particle size of oil droplets after thawing [µm] | 5.73 | 6.03 | 25.2 |
| | Rate of change in average particle size before and after freezing [%] | +17.0 | +23.2 | +250 |
| | Median diameter of oil droplets before freezing [µm] | 4.30 | 4.29 | 6.41 |
| | Median diameter of oil droplets after thawing [µm] | 5.13 | 4.95 | 13.9 |
| | Rate of change in median diameter before and after freezing [%] | +19.4 | +15.3 | +116 |

As apparent from the results shown in Tables 7 and 8, in the emulsion compositions according to the present invention, a change in the particle size of oil droplets was inhibited even in a temperature process where not only the discontinuous phase of each emulsion but also water constituting the continuous phase were frozen and melted (changed in state), and a stable emulsified state was maintained.

From these results, the oil-in-water emulsion compositions according to one embodiment of the present invention were found to have excellent freezing resistance.

### <Evaluation of Quality of Taste>

### (Evaluation Example 1)

Six research workers engaged in research and development, serving as panelists (taste panels), performed evaluation by a two-point comparison method at room temperature. The panelists were asked to choose the presence or absence of richness in a beverage containing the oil-in-water emulsion composition A1 against the below-described standard beverage at room temperature. It is noted here that the oil-in-water emulsion composition A1 according to Example 3 was used after being heat-sterilized at 121°C for 30 minutes, cooled to room temperature, and then stored refrigerated. This emulsion composition A1 was mixed with demineralized water to adjust the resulting beverage to have the oil-and-fat concentration shown in Table 9.

The particle size distribution and the pH of the standard beverage and those of the oil-in-water emulsion composition A1 were as shown in Table 9.

### (Standard Beverage)

A composition containing a sucrose fatty acid ester along with 1% of hydrogenated coconut oil as an oil-phase component was used as a standard beverage.

**Table 9**

| | | Standard beverage | Beverage containing emulsion composition A1 |
|---|---|---|---|
| Oil-and-fat content in beverage [%] | | 1 | 1 |
| Based on number | Average particle size of oil droplets [µm] | 0.121 | 2.15 |
| | Median diameter of oil droplets [µm] | 0.103 | 1.91 |
| Based on volume | Average particle size of oil droplets [µm] | 0.400 | 4.29 |
| | Median diameter of oil droplets [µm] | 0.327 | 3.78 |
| pH | | 7.23 | 6.46 |

### <Samples>

Two kinds of emulsions prepared by different emulsification methods: #1 (standard beverage) and #2 (beverage containing the emulsion composition A1)

### <Method>

Two-point comparison method (the panelists were given the two kinds of samples and asked to choose the presence or absence of richness in #2 against #1)

### <Panelists>

Taste panels, n = 6

### <Results>

Number of panelists answering that #2 had richness: 6
Number of panelists answering that #2 lacked richness: 0
Note) There was a significant difference at the 5% level.

### (Evaluation Example 2)

Six research workers engaged in research and development, serving as panelists (taste panels), performed evaluation by a ranking method at room temperature. The below-described sample #1 (standard beverage) and sample #2 (beverage containing the emulsion composition A1) had the respective formulations shown in Table 9 as in Evaluation 1.

As a sample #3 (beverage containing the protein particle aqueous dispersion A1), a beverage prepared by heat-sterilizing the protein particle aqueous dispersion A1 according to Example 2 at 121°C for 30 minutes, cooling this aqueous dispersion A1 to room temperature, storing the aqueous dispersion A1 refrigerated, and then 5-fold diluting the aqueous dispersion A1 with demineralized water (protein particle concentration: 1%, pH = 7.23) was used.

The sample #2 (beverage containing the emulsion composition A1) had a protein particle concentration of 0.95%.

### <Samples>

#1 (standard beverage), #2 (beverage containing the emulsion composition A1), and #3 (beverage containing the protein particle aqueous dispersion A1)

### <Method>

The panelists were given the three kinds of samples and asked to rank the samples based on the intensity of richness.

### <Panelists>

Taste panels, n = 6

### <Results>

The results are shown in Table 10.

**Table 10**

| | Sample | | |
|---|---|---|---|
| Panelist | #1 Standard beverage | #2 Beverage containing emulsion composition A1 | #3 Beverage containing protein particle aqueous dispersion A1 |
| No.1 | 3 | 1 | 2 |
| No.2 | 2 | 1 | 3 |
| No.3 | 3 | 1 | 2 |
| No.4 | 3 | 1 | 2 |
| No.5 | 2 | 1 | 2 |
| No.6 | 3 | 1 | 2 |
| Total rank | 16 | 6 | 13 |

From the results of Evaluation Example 1 and Evaluation Example 2, the emulsion compositions according to one embodiment of the present invention were found to have a significantly enhanced richness as compared to the standard beverage prepared using a surfactant.

### <Preparation of Beverages>

### (Evaluation 1) Coffee and Tea Beverages

The oil-in-water emulsion composition A1 (oil-and-fat concentration in whole composition: 5%) was heat-sterilized at 121°C, subsequently stored at 4°C, brought back to normal temperature, and then added to various beverages, followed by mixing. The oil-and-fat concentration in the composition after the mixing was 1%. Tables 11 and 12 show the results of measuring the pH at room temperature and the particle size distribution for each of the emulsion composition A1, the emulsion composition A1 after 30-minute heat sterilization at 121°C, and mixtures obtained by adding and mixing the emulsion composition A1, which had been heat-sterilized at 121°C for 30 minutes, with the respective beverages.

Table 11 shows the particle size distribution measurement results that were analyzed based on number, while Table 12 shows the particle size distribution measurement results that were analyzed based on volume.

The beverages used for mixing with the emulsion were prepared as follows.

A tea was prepared by adding a bag of AGF Professional Instant Tea manufactured by Ajinomoto AGF, Inc. to 1,000 g of hot water and mixing the resultant.

A coffee was prepared by mixing 1.5 parts of NESCAFE Gold Blend manufactured by Nestle Japan Ltd. and 0.05 parts of baking soda in 98.45 parts of hot water, and subsequently removing insoluble components by filter filtration.

A soup was prepared by diluting a bag (12 mL) of YAMAKI Dashi Broth (Katsuo, Soda-Katsuo, Kombu) manufactured by Yamaki Co., Ltd. with 150 g of hot water (prepared by heating demineralized water).

**Table 11**

| Based on number | | Type of beverage mixed with emulsion composition | | | |
|---|---|---|---|---|---|
| | | Deminerali zed water | Tea | Coffee | Soup |
| Physical properties of emulsion composition A1 (unsterilized) | Average particle size of oil droplets [µm] | 1.95 | 1.95 | 1.95 | 2.10 |
| | Median diameter of oil droplets [µm] | 1.75 | 1.75 | 1.75 | 1.90 |
| | pH | 7.20 | 7.20 | 7.20 | 7.25 |
| Physical properties of emulsion composition A1 (sterilized) | Average particle size of oil droplets [µm] | 2.09 | 2.09 | 2.09 | 2.16 |
| | Median diameter of oil droplets [µm] | 1.88 | 1.88 | 1.88 | 1.89 |
| | pH | 7.08 | 7.08 | 7.08 | 7.10 |
| Physical properties measured after addition and mixing of emulsion composition A1 with each beverage | Average particle size of oil droplets [µm] | 2.15 | 2.14 | 2.17 | 13.1 |
| | Median diameter of oil droplets [µm] | 1.91 | 1.91 | 1.93 | 12.1 |
| | pH | 7.23 | 6.77 | 6.69 | 6.12 |
| pH of each beverage mixed | | 5.62 | 5.44 | 6.52 | 5.02 |
| Rate of change in average size of oil droplets before and after mixing of emulsion composition A1 with each beverage [%] | | +2.87 | +2.39 | +3.83 | +506 |
| Rate of change in median diameter of oil droplets before and after mixing of emulsion composition A1 with each beverage [%] | | +1.60 | +1.60 | +2.66 | +540 |

**Table 12**

| Based on volume | | Type of beverage mixed with emulsion composition | | | |
|---|---|---|---|---|---|
| | | Deminerali zed water | Tea | Coffee | Soup |
| Physical properties of emulsion composition A1 (unsterilized) | Average particle size of oil droplets [µm] | 3.83 | 3.83 | 3.83 | 3.76 |
| | Median diameter of oil droplets [µm] | 3.24 | 3.24 | 3.24 | 3.30 |
| | pH | 7.20 | 7.20 | 7.20 | 7.25 |
| Physical properties of emulsion composition A1 (sterilized) | Average particle size of oil droplets [µm] | 3.95 | 3.95 | 3.95 | 4.68 |
| | Median diameter of oil droplets [µm] | 3.44 | 3.44 | 3.44 | 4.08 |
| | pH | 7.08 | 7.08 | 7.08 | 7.10 |
| Physical properties measured after addition and mixing of emulsion composition A1 with each beverage | Average particle size of oil droplets [µm] | 4.29 | 4.28 | 4.32 | 21.9 |
| | Median diameter of oil droplets [µm] | 3.78 | 3.69 | 3.78 | 20.1 |
| | pH | 7.23 | 6.77 | 6.69 | 6.12 |
| pH of each beverage mixed | | 5.62 | 5.44 | 6.52 | 5.02 |
| Rate of change in average size of oil droplets before and after mixing of emulsion composition A1 with each beverage [%] | | +8.61 | +8.35 | +9.73 | +368 |
| Rate of change in median diameter of oil droplets before and after mixing of emulsion composition A1 with each beverage [%] | | +9.88 | +7.27 | +9.88 | +393 |

As apparent from Tables 11 and 12, the emulsion composition according to one embodiment of the present invention was found to maintain such an emulsion stability that allows mixing of the emulsion composition with commercially available instant beverages as well.

<Evaluation of Storage Stability of Emulsion compositions and Beverages>

### (Evaluation 1: Example 5)

An oil-in-water emulsion composition A1', which was produced in the same manner as the oil-in-water emulsion composition A1 (oil-and-fat concentration in whole composition: 5%), was fractionated in a container, and then sterilized at 121°C for 30 minutes using an autoclave, whereby a sterilized oil-in-water emulsion composition A1' was prepared.

This sterilized oil-in-water emulsion composition A1' was stored at 4°C, 15°C, or 35°C for a prescribed period. Thereafter, the emulsion composition A1' was brought back to room temperature, and the particle size distribution of oil droplets and the pH were measured.

The results are shown in Tables 13 to 15.

**Table 13**

| Example 5 Emulsion composition A1' | | | Before steriliza tion | Storage period at 4°C after sterilization | | | |
|---|---|---|---|---|---|---|---|
| | | | | 0 day | 10 days | 14 days | 28 days |
| Based on number | Average particle size of oil droplets | [µm] | 2.14 | 2.06 | 1.97 | 1.95 | 2.00 |
| | Rate of change in average particle size of oil droplets | [%] | - | 0.00 | -4.37 | -5.34 | -2.91 |
| | Median diameter of oil droplets | [µm] | 1.87 | 1.84 | 1.77 | 1.73 | 1.77 |
| | Rate of change in median diameter of oil droplets | [%] | - | 0.00 | -3.80 | -5.98 | -3.80 |
| Based on volume | Average particle size of oil droplets | [µm] | 6.34 | 6.92 | 5.47 | 5.4 | 5.91 |
| | Rate of change in average particle size of oil droplets | [%] | - | 0.00 | -21.0 | -22.0 | -14.6 |
| | Median diameter of oil droplets | [µm] | 4.28 | 3.64 | 3.46 | 3.58 | 3.74 |
| | Rate of change in median diameter of oil droplets | [%] | - | 0.00 | -4.95 | -1.65 | +2.75 |
| pH | | | 7.29 | 7.06 | 7.13 | 7.12 | 7.07 |

**Table 14**

| Example 5 Emulsion composition A1' | | | Before steriliza tion | Storage period at 15°C after sterilization | | | |
|---|---|---|---|---|---|---|---|
| | | | | 0 day | 10 days | 14 days | 28 days |
| Based on number | Average particle size of oil droplets | [µm] | 2.14 | 2.06 | 1.87 | 1.85 | 1.85 |
| | Rate of change in average particle size of oil droplets | [%] | - | 0.00 | -9.22 | -10.2 | -10.2 |
| | Median diameter of oil droplets | [µm] | 1.87 | 1.84 | 1.66 | 1.61 | 1.64 |
| | Rate of change in median diameter of oil droplets | [%] | - | 0.00 | -9.78 | -12.5 | -10.9 |
| Based on volume | Average particle size of oil droplets | [µm] | 6.34 | 6.92 | 4.61 | 7.16 | 4.21 |
| | Rate of change in average particle size of oil droplets | [%] | - | 0.00 | -33.4 | +3.47 | -39.2 |
| | Median diameter of oil droplets | [µm] | 4.28 | 3.64 | 3.49 | 4.27 | 3.39 |
| | Rate of change in median diameter of oil droplets | [%] | - | 0.00 | -4.12 | + 17.3 | -6.87 |
| pH | | | 7.29 | 7.06 | 7.11 | 7.15 | 7.01 |

**Table 15**

| Example 5 Emulsion composition A1' | | | Before steriliza tion | Storage period at 35°C after sterilization | | | |
|---|---|---|---|---|---|---|---|
| | | | | 0 day | 10 days | 14 days | 28 days |
| Based on number | Average particle size of oil droplets | [µm] | 2.14 | 2.06 | 1.98 | 1.85 | 1.88 |
| | Rate of change in average particle size of oil droplets | [%] | - | 0.00 | -3.88 | -10.2 | -8.74 |
| | Median diameter of oil droplets | [µm] | 1.87 | 1.84 | 1.75 | 1.64 | 1.67 |
| | Rate of change in median diameter of oil droplets | [%] | - | 0.00 | -4.89 | -10.9 | -9.24 |
| Based on volume | Average particle size of oil droplets | [µm] | 6.34 | 6.92 | 5.00 | 5.49 | 4.55 |
| | Rate of change in average particle size of oil droplets | [%] | - | 0.00 | -27.7 | -20.7 | -34.2 |
| | Median diameter of oil droplets | [µm] | 4.28 | 3.64 | 3.65 | 3.39 | 3.44 |
| | Rate of change in median diameter of oil droplets | [%] | - | 0.00 | +0.28 | -6.87 | -5.49 |
| pH | | | 7.29 | 7.06 | 7.07 | 7.09 | 6.96 |

As apparent from Tables 13 to 15, it was found that, in the emulsion composition according to one embodiment of the present invention, oil phase separation does not occur even during long-term storage in a wide temperature range of 4 to 35°C, and a change in the particle size (average particle size and/or median diameter) of oil droplets is inhibited. Further, it was indicated that a pH change associated with deterioration of the oil-and-fat is inhibited as well.

Therefore, the emulsion composition according to one embodiment of the present invention can be said to have not only a long-term storage stability, but also such a stability that withstands the transport at normal temperature without being refrigerated. The emulsion composition according to one embodiment of the present invention is presumed to be a stable emulsion composition that also withstands the temperature changes during transport.

This can lead to a reduction in power consumption and contribute to cost reduction since it is longer necessary to finely control the post-production storage temperature and the transport temperature to be low. In addition, the emulsion composition according to one embodiment of the present invention is advantageous in that it can be subjected to a processing that requires temperature control, such as freezing, refrigeration, and heating, at the transport destination.

### (Evaluation 2: Examples 6 and 7)

An oil-in-water emulsion composition A1", which was produced in the same manner as the oil-in-water emulsion composition A1 (oil-and-fat concentration in whole composition: 5%), was added to various beverages (demineralized water and tea), followed by mixing. The oil-and-fat concentration in the composition after the mixing was 1%.

A beverage obtained by mixing the emulsion composition A1" with demineralized water and a beverage obtained by mixing the emulsion composition A1" with tea are hereinafter referred to as "water beverage" and "tea beverage", respectively.

The water beverage and the tea beverage were each fractionated in a container and then sterilized at 121°C for 30 minutes using an autoclave to prepare sterilized beverages.

It is noted here that the tea mixed with the emulsion was prepared in the same manner as described above in the section of <Preparation of Beverages>.

The sterilized beverages were stored at 4°C or 35°C for a prescribed period. Thereafter, the beverages were brought back to room temperature, and the particle size distribution of oil droplets and the pH were measured.

The results are shown in Tables 16 and 17.

**Table 16**

| Example 6 Mixed beverage of emulsion composition A1" and demineralized water | | | Before sterilizat ion | Storage period at 4°C after sterilization | | Storage period at 35°C after sterilization | | | |
|---|---|---|---|---|---|---|---|---|---|
| | | | | 0 day | 28 days | 0 day | 10 days | 14 days | 28 days |
| Based on number | Average particle size of oil droplets | [µm] | 2.06 | 2.14 | 2.02 | 2.06 | 1.98 | 1.85 | 1.88 |
| | Rate of change in average particle size of oil droplets | [%] | - | 0.00 | -5.61 | 0.00 | -3.88 | -10.2 | -8.74 |
| | Median diameter of oil droplets | [µm] | 1.83 | 1.90 | 1.78 | 1.84 | 1.75 | 1.64 | 1.67 |
| | Rate of change in median diameter of oil droplets | [%] | - | 0.00 | -6.32 | 0.00 | -4.89 | -10.9 | -9.24 |
| Based on volume | Average particle size of oil droplets | [µm] | 4.27 | 4.49 | 4.44 | 4.49 | 4.00 | 4.05 | 3.68 |
| | Rate of change in average particle size of oil droplets | [%] | - | 0.00 | -1.11 | 0.00 | -10.9 | -9.80 | -18.0 |
| | Median diameter of oil droplets | [µm] | 3.65 | 3.87 | 3.70 | 3.87 | 3.41 | 3.46 | 3.21 |
| | Rate of change in median diameter of oil droplets | [%] | - | 0.00 | -4.39 | 0.00 | -11.9 | -10.6 | -17.1 |
| pH | | | 7.45 | 7.16 | 7.23 | 7.16 | 7.29 | 7.25 | 7.04 |

**Table 17**

| Example 7 Mixed beverage of emulsion composition A1" and tea | | | Before sterilizat ion | Storage period at 4°C after sterilization | | Storage period at 35°C after sterilization | | | |
|---|---|---|---|---|---|---|---|---|---|
| | | | | 0 day | 28 days | 0 day | 10 days | 14 days | 28 days |
| Based on number | Average particle size of oil droplets | [µm] | 2.06 | 2.07 | 1.97 | 2.07 | 1.94 | 1.94 | 1.88 |
| | Rate of change in average particle size oil droplets | [%] | - | 0.00 | -4.83 | 0.00 | -6.28 | -6.28 | -9.18 |
| | Median diameter of oil droplets | [µm] | 1.83 | 1.83 | 1.77 | 1.83 | 1.72 | 1.74 | 1.70 |
| | Rate of change in median diameter of oil droplets | [%] | - | 0.00 | -3.28 | 0.00 | -6.01 | -4.92 | -7.10 |
| Based on volume | Average particle size of oil droplets | [µm] | 4.03 | 4.97 | 3.72 | 4.97 | 6.78 | 3.82 | 3.51 |
| | Rate of change in average particle size of oil droplets | [%] | - | 0.00 | -25.2 | 0.00 | + 36.4 | -23.1 | -29.4 |
| | Median diameter of oil droplets | [µm] | 3.52 | 3.91 | 3.23 | 3.91 | 3.59 | 3.25 | 3.00 |
| | Rate of change in median diameter of oil droplets | [%] | - | 0.00 | -17.4 | 0.00 | -8.18 | -16.9 | -23.3 |
| pH | | | 6.86 | 6.43 | 6.39 | 6.43 | 6.32 | 6.29 | 6.06 |

As apparent from Tables 16 and 17, it was found that, in the emulsion composition according to one embodiment of the present invention, oil phase separation does not occur even when an operation equivalent to food heat sterilization is performed after the preparation of a beverage, and a change in the particle size (average particle size and/or median diameter) of oil droplets is inhibited. Therefore, a beverage containing the emulsion composition according to one embodiment of the present invention has excellent emulsion stability, and thus can be said to be a stable beverage that has a long-term storage stability even after its production (including a sterilization step) and withstands the temperature changes during transport. The beverage also has such a stability that withstands the transport at normal temperature without being refrigerated, and can be controlled (by freezing, refrigeration, or heating) to have a temperature that conforms to the sales format at the transport destination.

## Claims

1. A protein, satisfying one of the following conditions (A) to (C) and being partially in the state of particles when made into an aqueous dispersion:
(A): having at least one peak in a region of a molecular weight of 66,000 or more in terms of polyethylene glycol in a gel filtration chromatography elution curve;
(B): having a weight-average molecular weight of more than 27,000 in terms of polyethylene glycol as measured by gel filtration chromatography; and
(C): having a number-average molecular weight of more than 3,500 in terms of polyethylene glycol as measured by gel filtration chromatography.

2. The protein according to claim 1, satisfying at least two of the conditions (A) to (C).

3. The protein according to claim 1 or 2, wherein a raw material protein constituting the protein is a plant protein.

4. The protein according to claim 3, wherein the plant protein is a protein derived from a Fabaceae plant.

5. The protein according to claim 4, wherein the Fabaceae plant is a *Phaseolus* plant, a *Cicer* plant, a *Pisum* plant, a *Lens* plant, or a *Lupinus* plant.

6. The protein according to any one of claims 1 to 5, wherein the particles in the aqueous dispersion have an average particle size of 0.005 µm to 10 µm.

7. A protein particle dispersion, comprising the protein according to any one of claims 1 to 6.

8. A method of producing the protein according to any one of claims 1 to 6, the method comprising the following steps (1) and (2):
Step (1): the step of mixing water and a raw material protein to prepare a mixture; and
Step (2): the step of heat-treating the mixture at 60°C to 200°C for 1 second to 120 minutes.

9. A method of producing a protein, the method comprising the following steps (1) and (2') and the protein being partially in the state of particles when made into an aqueous dispersion,
wherein the below-described raw material protein is a plant protein:
Step (1): the step of mixing water and a raw material protein to prepare a mixture; and
Step (2'): the step of heat-treating the mixture at 60°C to 200°C for 1 second to 120 minutes under a pressure other than normal pressure.

10. An emulsion composition, comprising water, an oil-and-fat, and a protein,
wherein
the protein exists in an interface between the water and the oil-and-fat, and
the protein is the protein according to any one of claims 1 to 6.

11. The emulsion composition according to claim 10, wherein the oil-and-fat is an edible oil-and-fat.

12. The emulsion composition according to claim 10 or 11, which is an oil-in-water emulsion composition.

13. A method of producing an emulsion composition, the method comprising:
a step of dispersing the protein according to any one of claims 1 to 6 in water to obtain an aqueous dispersion of protein particles; and
a step of mixing the thus obtained aqueous dispersion of protein particles with an oil-and-fat, and stirring the resulting mixture.

14. A method of producing an emulsion composition, the method comprising:
a step of dispersing the protein according to any one of claims 1 to 6 in an oil-and-fat to obtain an oil-and-fat dispersion of protein particles; and
a step of mixing the thus obtained oil-and-fat dispersion of protein particles with water, and stirring the resulting mixture.

15. The emulsion composition according to any one of claims 10 to 12, which is for a food.

16. A food, comprising the protein according to any one of claims 1 to 6.

17. A food, comprising the emulsion composition according to any one of claims 10 to 12.

18. An animal-derived food substitute, comprising the protein according to any one of claims 1 to **6.**

19. An animal-derived food substitute, comprising the emulsion composition according to any one of claims 10 to 12.

20. A milk substitute, comprising the protein according to any one of claims 1 to **6.**

21. A milk substitute, comprising the emulsion composition according to any one of claims 10 to 12.

22. A pharmaceutical product, comprising the protein according to any one of claims 1 to 6.

23. A pharmaceutical product, comprising the emulsion composition according to any one of claims 10 to 12.

24. A cosmetic product, comprising the protein according to any one of claims 1 to 6.

25. A cosmetic product, comprising the emulsion composition according to any one of claims 10 to 12.

26. A personal care product, comprising the protein according to any one of claims 1 to 6.

27. A personal care product, comprising the emulsion composition according to any one of claims 10 to 12.

28. Protein particles, satisfying one of the following conditions (A) to (C) when made into an aqueous dispersion:
(A): having at least one peak in a region of a molecular weight of 66,000 or more in terms of polyethylene glycol in a gel filtration chromatography elution curve;
(B): having a weight-average molecular weight of more than 27,000 in terms of polyethylene glycol as measured by gel filtration chromatography; and
(C): having a number-average molecular weight of more than 3,500 in terms of polyethylene glycol as measured by gel filtration chromatography.
